# EUROPEAN PATENT SPECIFICATION

(11) **EP 3 579 892 B1**
(45) Date of publication and mention of the grant of the patent: **30.10.2024**
(21) Application number: 18751317.1
(22) Date of filing: 09.02.2018
(51) Int. Cl.: A61M 1/06, A61H 15/00

(54) **BREAST MILK EXPRESSION APPARATUS AND SYSTEM**
VORRICHTUNG UND SYSTEM ZUR EXPRESSION VON MUTTERMILCH
APPAREIL ET SYSTÈME D'EXPRESSION DE LAIT MATERNEL

(30) Priority: 13.02.2017 US 201715431644; 05.12.2017 US 201762594857 P
(43) Date of publication of application: 18.12.2019
(73) Proprietor: Imalac, Inc., Miami, FL 33156 (US)
(72) Inventor: SABLOTSKY, Noreen Gordon, Miami FL 33158 (US); KISH, Rachael Sablotsky, Miami FL 33158 (US); SABLOTSKY, Kathryn Garriott, Charleston SC 29492 (US); ARP, Scott R., Miami FL 33165 (US); BALES, William Thomas, Miami FL 33143 (US); GRATA, Paul John, Miami Lakes FL 33014 (US); TOMLIN, Damian H., Coral Springs FL 33065 (US); GAMEZ, Victor M., Fort Lauderdale FL 33305 (US); ALVAREZ, Lisandro Rivera, Miramar FL 33027 (US)
(74) Representative: Murgitroyd & Company
(86) International application number: PCT/US2018/017624
(87) International publication number: WO 2018/148546

(56) References cited:
- US-A- 5 514 166
- US-A1- 2006 106 334
- US-A1- 2006 106 334
- US-A1- 2014 378 946
- US-A1- 2015 065 994
- US-A1- 2017 112 983
- US-A1- 2017 112 983

## Description

### BACKGROUND

### 1. Field

This disclosure relates to a breast milk expression apparatus and system.

### 2. State of the Art

Women's breasts are made of specialized tissues that produce milk. This includes glandular as well as fatty tissues. The milk-producing part of the breast is organized into 15 to 20 sections, called lobes. Within each lobe are smaller structures, called lobules, where milk is produced. The milk travels through a network of tiny tubes called ducts. The ducts connect and come together into larger ducts, which eventually exit the skin in the nipple. There is a considerable body of evidence in the literature on the proven and potential benefits of breast milk expression during lactation. These benefits include, but are not limited to: clearing clogged milk ducts, assisting milk to flow more freely through the ducts resulting in a decrease in time required and increase in quantity of milk expressed during any given single pumping event, creation of subjective physical pleasure and decrease in generalized breast pain, increase in the positive caloric nutritional value of the breast milk resulting from more complete emptying of all lactation tissue. Hand expression is often used as a viable method for milk extraction, and is more commonly recommended by leading lactation experts as a simultaneous adjunct to breast pumping.

An example of a breast milk expression system and method is described in US2006/0106334, wherein the system comprises various mechanisms for stimulating the breast to aid in lactation. An example of a massaging lactation assistive device is described in US2017/112983, which includes electrical or mechanical components to stimulate a breast An example of a device and method for supporting a breast shield of a breast pump in a "hands-free" manner is described in US5514166.

### SUMMARY

As will be appreciated from the following description, in accordance with at least one aspect, a breast milk expression apparatus and system can replace manual (by hand) milk extraction.

The present invention provides a breast milk expression apparatus as defined in the appended claims. According to one embodiment, the breast milk expression apparatus includes an outer shell, and a plurality of circumferentially spaced inner pads coupled to the outer shell. The pads are configured to engage a breast of the user and configured for radial displacement. The breast milk expression apparatus further includes a compression unit coupled to the plurality of pads. The compression unit is configured to cyclically displace the pads radially inwardly against the breast to cause compression of the breast and, following compression of the breast, to permit displacement of the pads radially outwardly from the breast to allow for decompression of the breast

The outer shell, which may be manufactured in multiple sizes, may have an inner surface that is generally concave. The inner surface may be convex near an axillary part of the breast The sizes of the outer shell may correspond to bra cup sizes. The inner surface may be configured to engage a maximum possible volume of a user's breast tissue. The outer shell may define a central opening about which the plurality of inner pads are circumferentially spaced. The central opening may be dimensioned to receive a breastshield therethrough for engagement with the user's breast Milk expressed during use of the breast milk expression apparatus may be directed by the breastshield away from the breast, such as to a collection bottle.

The pads that are coupled to the outer shell may be variously shaped and configured to engage the outer surface of a user's breast for breast manipulation to facilitate milk expression. Also, at least one of the pads may be heated.

The breast milk expression apparatus may include an outer cover that may cover the outer shell. The outer cover may function to protect the outer shell and/or the compression unit and may also provide a desired aesthetic look. The breast milk expression apparatus may also include an inner liner to cover the pads coupled to the outer shell. The inner liner may provide a comfort barrier between the skin of a user's breast and the pads. In one embodiment, the outer cover and the inner liner are coupled together to form an encasement for the breast milk expression apparatus. The outer cover, inner liner, and the encasement may be removable from the breast milk expression apparatus.

In one embodiment of the breast milk expression apparatus, the compression unit includes a cable banded about and through the plurality of pads, and a drive unit coupled to the cable and the outer shell. The drive unit is configured to tighten the cable about the pads to cause the pads to compress the breast and is configured to subsequently loosen the cable about the pads, permitting the breast to expand and decompress.

Each pad may define a circumferential channel through which the cable extends. The circumferential channel may permit relative movement between the cable and the pad. The drive unit may include an electric motor configured to drive a transmission system to alternate tension in the cable. In one embodiment, the drive unit is configured to receive at least one of power and control setting signals from a controller. The drive unit may vary one or more of a pressure applied by the pads to the breast, a duration of compression and decompression, and a heating level of the pads.

At least one of the pads and the outer shell may be made of one or more polymeric materials, such as ABS, nylon, polyurethane, and silicone, and may have a Shore hardness of 10A to 90D.

According to another aspect, further details of which are provided below, a breast milk expression system includes at least one breast milk expression apparatus configured to cyclically compress and allow decompression of a breast of a user, at least one strap coupled to the breast milk expression apparatus and configured to position the breast milk expression apparatus on a user's breast, and a controller configured to control the operation of breast milk expression apparatus. The breast milk expression apparatus of the breast milk expression system may be any of the previously described breast milk expression apparatuses. The breast milk expression system may be used for milk expression of one or both breasts of a user, individually or simultaneously.

The controller may be configured to control one or more of breast manipulation pressure, temperature, and manipulation speed. The controller may be wired or wireless. The breast milk expression apparatus of the system may include a power supply to power at least one of the breast milk expression apparatuses and the controller. The controller may include a power supply to power at least one of the controller and one or both of the breast milk expression apparatuses. The controller and/or the breast milk expression apparatus(es) may be operated through a computing device, such as a smart phone, tablet computer, smart watch, or other computing device communicatively coupled to the controller and/or the breast milk expression apparatus(es).

In one embodiment of the system, two breast milk expression apparatuses and a set of straps are arranged so that they may be worn like a bra, such as any type of bra known in the art The straps may be integrated or otherwise attached to the aforementioned cover and/or liner. The straps are adjustable for personalized fit to the wearer. The straps may permit separation of the two breast milk expression apparatuses from one another so that, for example, the straps can be rearranged to wear one breast milk expression apparatus independently of the other breast milk expression apparatus. The breast milk expression apparatuses may be removable from the straps.

In one embodiment, a breast milk expression system includes a separate bra for supporting breasts of a user and at least one breast milk expression apparatus coupled to the bra and positioned between the bra and the breast of the user. The breast milk expression apparatus may be removably coupled to the bra so that the breast milk expression apparatus can be decoupled from the bra so that that bra can be used without the breast milk expression apparatus. The bra may be any type of bra known in the art. The bra may be integrated with or otherwise attached to the aforementioned cover and/or liner.

According one another aspect, a breast milk expression apparatus includes a compression pad configured to substantially surround a breast of a user, and a string coupled to the compression pad and extending circumferentially around an outer surface of the compression pad. The string is configured to slide relative to the outer compression pad. The apparatus also includes a string tensioning unit coupled to at least one end of the string, the string tensioning unit configured to apply tension to the string to cyclically displace the compression pad radially inwardly against the breast to cause compression of the breast and, following compression of the breast, permitting the breast to expand and decompress. According to one embodiment, the breast milk expression apparatus may also include a plurality of beads fixed to the compression pad and through which the string extends. The beads are circumferentially spaced from one another around the outer surface of the compression pad, and the string is configured to slide through the beads.

According to one embodiment, the string tensioning unit includes a motor and a transmission element driven by the motor, the transmission element coupled to the string. The transmission element may include a rack and pinion gear.

According to one embodiment, the breast milk expression apparatus includes a string routing unit for routing at least one end of the strings from the compression pad to the string tensioning unit

According to one embodiment, the compression pad is configured to connect to a bra that is configured to support the compression pad in engagement with the breast of the user. Also, in one embodiment, the string is configured to automatically radially expand in response to the expansion of the breast following compression of the breast.

According to the claimed invention, the compression pad has a tapered outer surface and extends longitudinally from a larger diameter first end to a smaller diameter second end, and the breast milk expression apparatus may include a plurality of strings including a first string located at a first longitudinal position and a second string located at a second longitudinal position that is longitudinally spaced from the first longitudinal position and closer to the second end. If desired, three, four, or even more strings may be utilized. Each of the strings is coupled to the compression pad and extends circumferentially around an outer surface of the compression pad, and each string is configured to slide relative to the outer compression pad. The string tensioning unit may include a motor and a transmission element driven by the motor, the transmission element coupled to the plurality of strings and configured to sequentially tension the strings.. In one embodiment, the transmission element includes a rack connected to the first string and a pinion gear engaged with the rack and driven by the motor. In one embodiment, the second string is connected to a pull member and the rack includes an engagement member configured to engage the pull member after a predetermined amount of relative translation between the rack and the pull member. In one embodiment, the transmission element is configured to translate the strings linearly relative to one another. According to the claimed invention, the transmission element is configured to apply tension to the first string for a longer duration than the second string.

According to another aspect, a breast milk expression system includes at least one breast milk expression apparatus configured to cyclically compress a breast of a user and, following compression, permit expansion and decompression of the breast, and at least one bra coupled to the breast milk expression apparatus and configured to position the breast milk expression apparatus on a user's breast.

In one embodiment, the bra has an inner layer and an outer layer and at least a portion of the breast milk expression apparatus is configured to be connected to the bra between the inner and outer layer. In another embodiment, at least a portion of the breast milk expression apparatus is configured to be connected to the bra under the inner layer. In one embodiment, the breast milk expression apparatus includes a compression pad configured to substantially surround a breast of a user, and the compression pad is connected to the inner layer of the bra. Also, the breast milk expression apparatus includes a string coupled to the compression pad and extends circumferentially around an outer surface of the compression pad, and the string is configured to slide relative to the outer compression pad. Further, the breast milk expression apparatus includes a string tensioning unit coupled to at least one end of the string. The string tensioning unit is configured to apply tension to the string to cyclically displace the compression pad radially inwardly against the breast to cause compression of the breast and, following compression of the breast, permitting the breast to expand and decompress.

In one embodiment, the compression pad includes a removable connector configured to removably connect to a connector secured to an inner layer of the bra located about a base of a cup of the bra. In one embodiment, the connector of the compression pad and the connector of the inner layer of the bra slide together. In one embodiment, the connector of the compression pad and the connector of the inner layer of the bra are portions of a zipper closure. In one embodiment, the connector of the inner layer of the bra extends about at least portion of a lower half of the base of the cup.

### BRIEF DESCRIPTION OF THE DRAWINGS

Figs. 1A and 1B show an embodiment of a breast milk expression system arranged on a torso.
Fig. 1C shows an embodiment of a breast milk expression system that includes a bra and a breast milk expression apparatus of the system of Figs. 1A and 1B.
Fig. 2 shows a top perspective view of a breast milk expression apparatus of the system of Figs. 1A and 1B along with a breastshield.
Fig. 3 illustrates a top view of one of the breast milk expression apparatuses shown in Fig. 1A.
Fig. 4 illustrates the breast milk expression apparatus shown in Fig. 3 with an outer shell omitted to show an arrangement of pads of the breast milk expression apparatus located relative to a breast of the user.
Fig. 5 is a view of the pads of Fig. 4 along section 5-5 shown in Fig. 4, and with the breast omitted.
Fig. 6 is an enlarged view of a drive unit of one of the breast milk expression apparatuses shown in Fig. 1A
Fig. 7 shows a perspective view of another embodiment of a breast milk expression apparatus.
Fig. 7A shows an exploded view of a portion of the breast milk expression apparatus of Fig. 7.
Fig. 8 shows a view of the breast milk expression apparatus of Fig. 7 from an inner side thereof.
Fig. 9 shows the breast milk expression apparatus of Fig. 8 with pads removed therefrom to show details of an outer shell of the breast milk expression apparatus.
Fig. 10 shows the breast milk expression apparatus of Fig. 7 positioned on a breast.
Fig. 11a is a front view of another embodiment of a breast milk expression system.
Fig. 11b shows an assembly view of a breast milk expression apparatus of the system shown in Fig. 11a.
Fig. 12a show a front view of the bra shown in Fig. 11a.
Fig. 12b shows the bra of Fig. 12a from the rear and a side.
Fig. 12c shows the bra of Figs. 12a and 12b with cups partially folded down.
Fig. 12d is a front and side view of the bra shown in Fig. 1c with the cups folded further down.
Figs. 13a and 13b show details of one connection arrangement for connecting a compression pad of the breast milk expression apparatus shown in Figs. 11a and 11b to the bra shown in Figs. 11a, 11b, and 12a to 12d.
Figs. 13c to 13e show details of a sequence of attaching the expression apparatus of Fig. 11a to the bra shown in Figs. 11a, 11b, and 12a to 12d using the connection arrangement shown in Figs. 13a and 13b.
Fig. 14 shows details of another connection arrangement for connecting a compression pad of the breast milk expression apparatus shown in Figs. 11a and 11b to the bra shown in Figs. 11a, 11b, and 12a to 12d.
Fig. 15 shows a detailed view of the breast milk expression apparatus shown in Figs. 11a and 11b.
Fig. 16a shows a side view of another embodiment of the breast milk expression apparatus.
Fig. 16b is a front view of the breast milk expression apparatus shown in Fig. 16a.
Figs. 17a to 17c show details of the operation of the breast milk expression apparatus shown in Figs. 16a and 16b.
Fig. 18a shows another embodiment of a breast milk expression system.
Fig. 18b shows the breast milk expression system of Fig. 18a with a cover of a module removed to show detail inside the module.
Figs. 19a to 19c shows details of another connection arrangement for connecting a compression pad of the breast milk expression apparatus shown in Figs. 11a and 11b to the bra shown in Figs. 11a, 11b, and 12a to 12d.

### DETAILED DESCRIPTION OF THE PREFERRED EMBODIMENTS

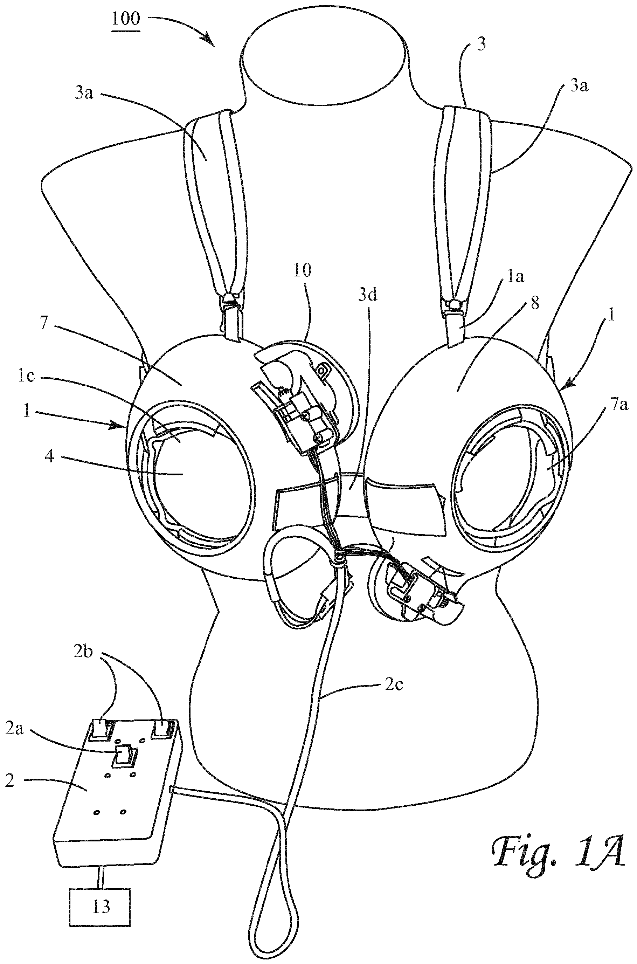

Fig. 1A shows one embodiment of a breast milk expression system 100 that includes two breast milk expression apparatuses 1 (hereinafter referred to as "expression apparatuses"), a controller 2 coupled to the expression apparatuses 1 via a power and control cords 2c, and a plurality of straps 3 for arranging the expression apparatuses 1 on the breasts 4 of a user and for connecting the expression apparatuses 1 together. Preferably, the straps 3 and expression apparatuses 1 are arranged and used like a bra to facilitate hands-free operation of one or both of the expression apparatuses 1. For example, the straps 3 may support the expression apparatuses 1 over the breasts 4 without a user holding the expression apparatuses 1 in place, either when the expression apparatuses are on or off. Two expression apparatuses 1 are shown included with the system 100 in Fig. 1A, where one expression apparatus 1 corresponds to each breast While two expression apparatuses 1 are shown, they may operate independently or together (e.g., simultaneously). Also, in other embodiments of the system, only one expression apparatus 1 may be included and a user may alternate its use from one breast to another as desired. Each expression apparatus 1 is configured to manipulate the tissue of the corresponding breast 4 based on a control input from the controller 2, as described in greater detail below. The milk expression system 100 can be used before, during, and/or after breast pumping or nursing to facilitate lactation and improve milk production and flow.

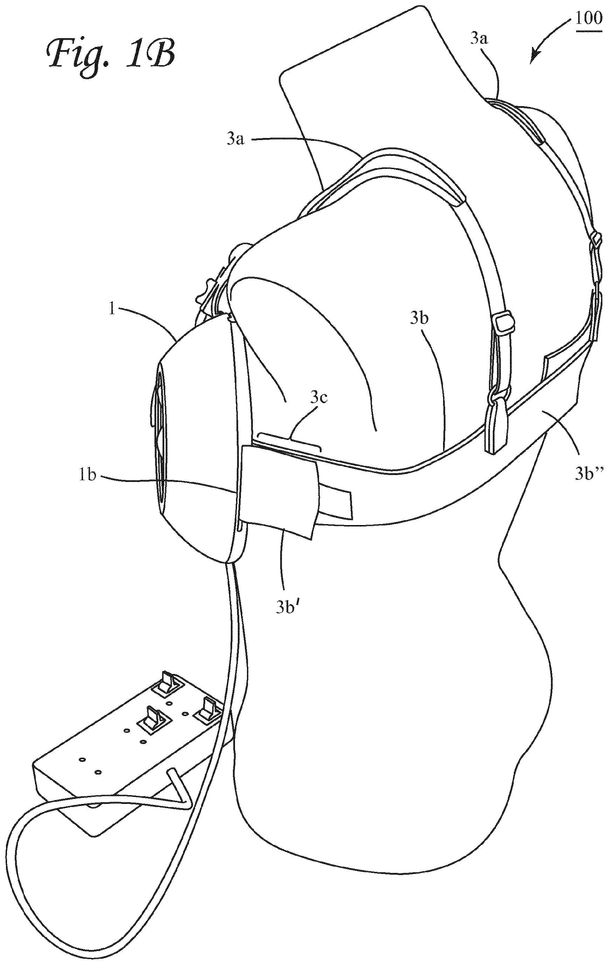

The plurality of straps 3 includes adjustable top straps 3a, each of which is attached at a top location 1a of the expression apparatuses 1. Each top strap 3a is configured to extend upward from a corresponding expression apparatus 1 and go over the shoulders and down the back of the user to connect to a corresponding adjustable side or back strap 3b (hereinafter referred to as a "side strap"), as shown in further detail in Fig. 1B. The top straps 3a may be padded for user comfort. Each side strap 3b has a first end 3b' coupled to an outer, side location 1b (Fig. 1B) of a corresponding expression apparatus 1, and has a second end 3b" opposite the first end 3b'. The side strap 3b extends from the expression apparatus 1 around the side of the user's torso towards the center of the user's back, where the second ends 3b" of each side strap 3b may be fastened together. The second ends 3b" of the side straps 3b may include hooks and loops (not shown) for fastening the second ends 3b" together. Also, the length of the side straps 3b may be adjustable at either end 3b', 3b" thereof. For example, in the embodiment shown in Fig. 1B, each expression apparatus 1 may have a loop or slot on its side 1b through which a first end 3b' of the side strap 3b can be wrapped. The first end 3b' may have hooks or loops and an overlapping portion 3c of the strap 3b may have mating hooks or loops to adjust the length of the side strap 3b.

In the shown embodiment, a center strap 3d (Fig. 1A) between the user's breasts 4, couples the expression apparatuses 1 together. The expression apparatuses 1 may have loops or slots on inner side locations 1d of the expression apparatuses 1 for coupling ends of the center strap 3d thereto, similar to the loops or slots on the outer side locations 1b of the expression apparatuses 1 for coupling side straps 3b. The length of the center strap 3d may also be adjustable to adjust the spacing between the expression apparatuses 1. The center strap 3d may permit decoupling or separation of the two expression apparatuses 1 from one another and from the center strap 3d. For example, the ends of the strap 3d may be detachably connected to the side locations 1d of respective expression apparatuses 1. Also, the center strap 3d may be formed as two parts that are connectable at respective ends between the side locations 1d, in the same manner that side straps 3b connect at ends 3b" between side locations 1b of expression apparatuses 1.

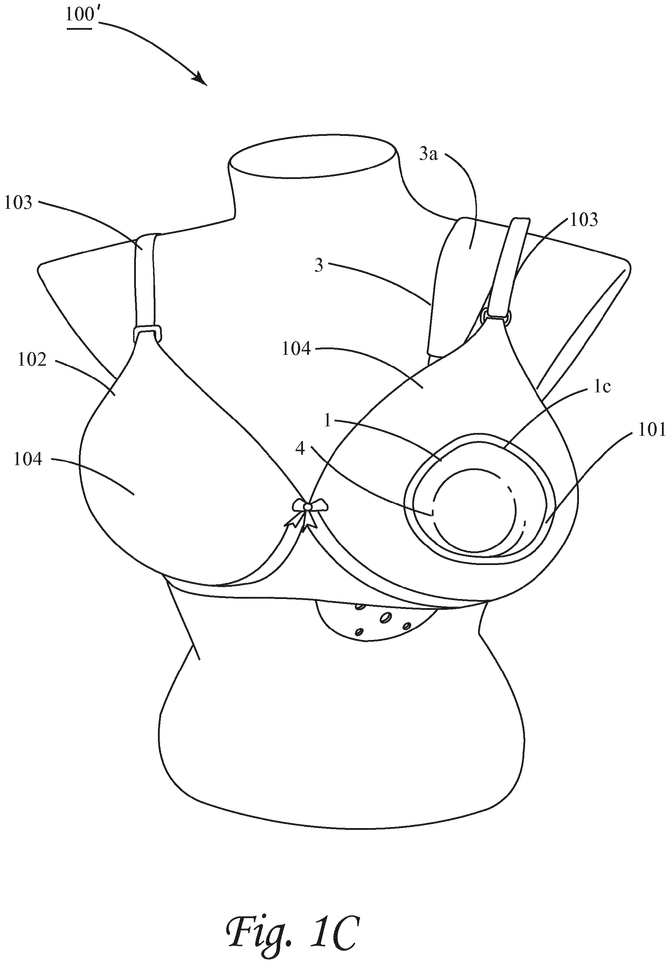

While the system 100 shows straps 3 and expression apparatuses 1 worn like a bra, in one embodiment shown in Fig. 1C, a system 100' includes a separate bra 102 and atleastone breast expression apparatus 1 (partially covered by the bra 102 in Fig. 1C) coupled to the bra 102 and positioned between the bra 102 and the breast 4. The bra 102 may have breast cups 104 attached to one or more straps 103, and one breast expression apparatus 1 may be removably coupled to an inside of a corresponding breast cup 104 so that the expression apparatus 1 can be positioned between the user's breast 4 and the cup 104 of the bra 102. The expression apparatus 1 may be coupled to the breast cup 104 using one or more fasteners or simply held in position by a friction or interference fit between the breast cup 104 and the expression apparatus 1. In such an embodiment, the expression apparatus 1 can be decoupled from the 102 bra (e.g., the cup 104 of the bra 102) so that that bra 102 can be used without the expression apparatus 1.

One or both of the cups 104 of the bra 102 may be solid (e.g., left cup in Fig. 1C) or may have at least one opening 101 (e.g. right cup in Fig. 1C). In the example shown in Fig. 1C, the opening 101 is aligned with opening 1c of the expression apparatus 1. The opening 101 may be sized to be at least as large as the opening 1c.

Although Fig. 1C shows one example bra, the aforementioned bras may be any type of bra known in the art, including, without limitation, a halter bra, a bandeau bra, a balconette bra, a contour or molded cup bra, a convertible bra, a demi cup bra, a full cup bra, a mastectomy bra, a maternity and nursing bra, a minimizer bra, a padded bra, a plunge bra, a push-up bra, a racerback bra, a shelf bra, a sports bra, a strapless bra, a T-shirt bra, and a U-plunge bra.

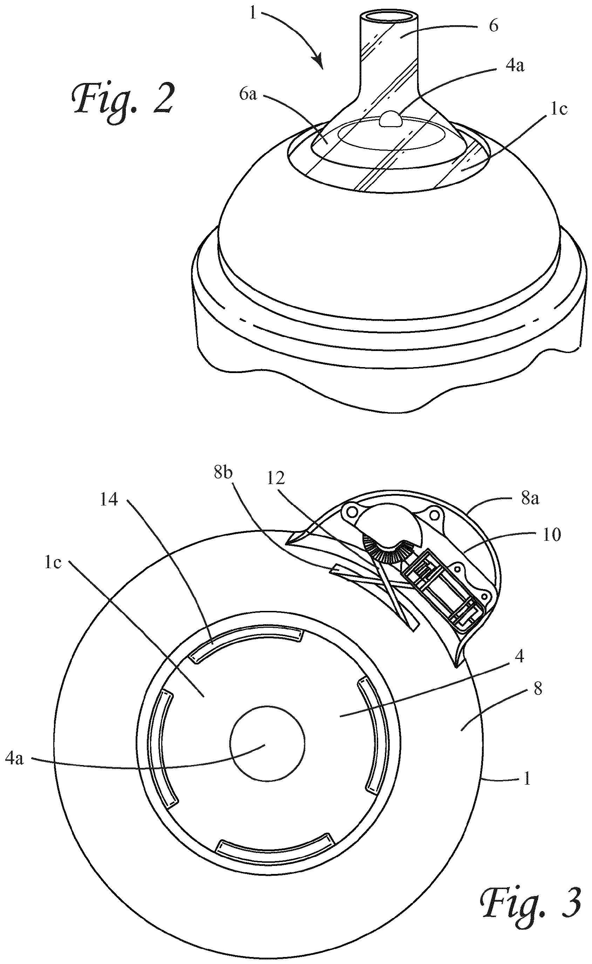

In use, the expression apparatuses 1 of the breast milk expression system 100 are positioned over and about the breasts 4. As shown in Figs. 1A to 3, the expression apparatuses 1 define a central opening 1c in which a nipple 4a of the breast 4 can be positioned. The central opening 1c provides access for a user to position a breastshield 6 (Fig. 2) within the opening 1c over the nipple 4a to facilitate milk collection (e.g., into a bottle or by breast pumping) while using the system 100. Preferably, the diameter of the central opening 1c is large enough to accommodate a range of sizes of breastshields 6 being marketed. For example, the diameter of the central opening 1c may be large enough to accommodate a breastshield having a diameter of 36 mm.

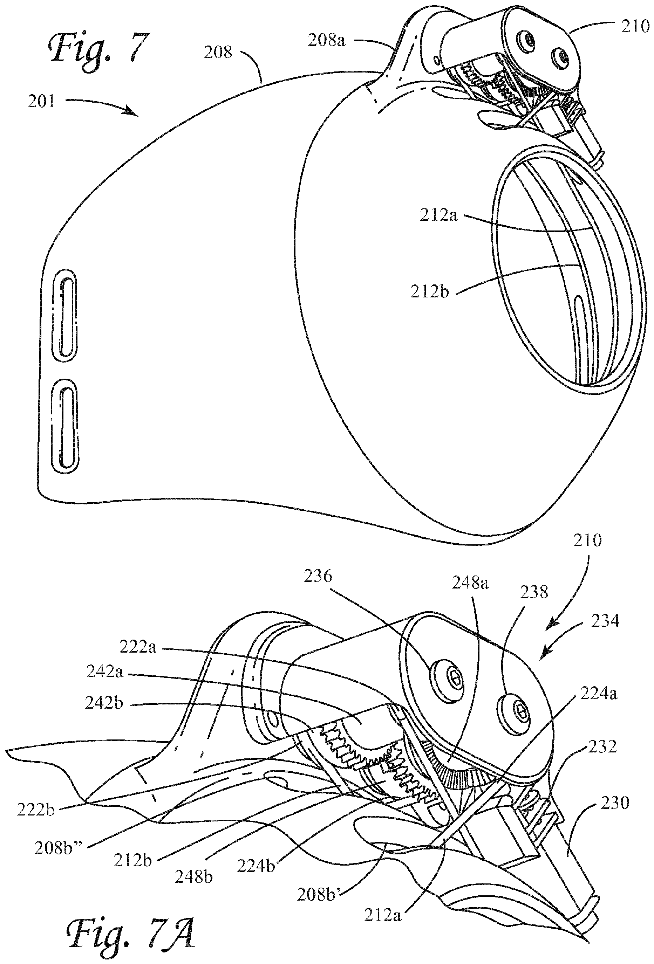

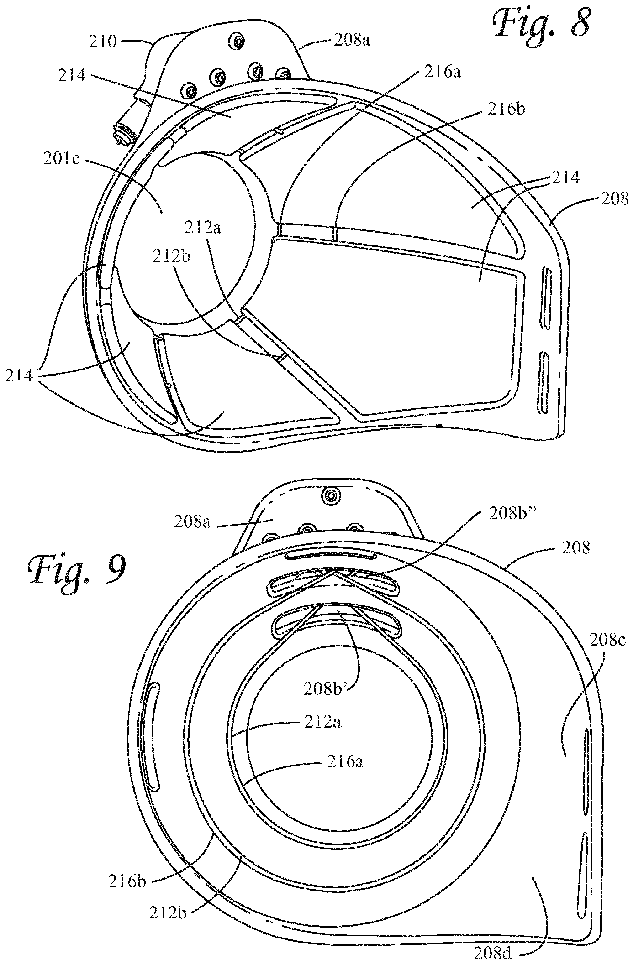

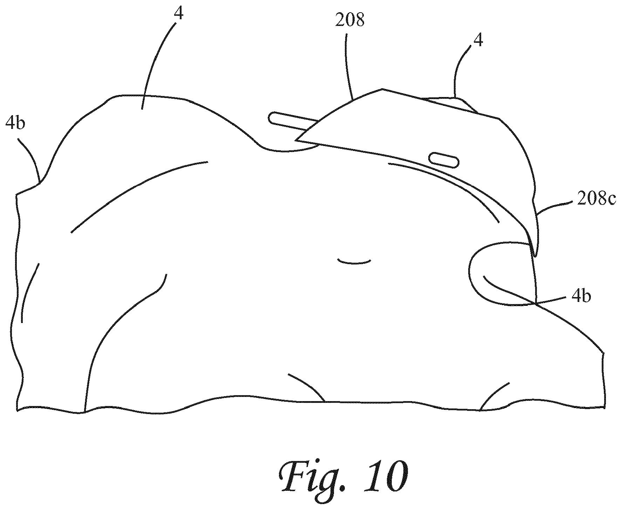

In the breast milk expression system 100, each expression apparatus 1 is functionally identical. Fig. 3 shows a view of one of the expression apparatuses 1 shown in Fig. 1A. The expression apparatus 1 has an outer shell 8, which may be made of ABS, nylon, polyurethane, or silicone having a Shore hardness of about 10A to 90D. The outer shell 8 has an inner form that is generally concave to conform to the contour of a plurality of pads 14, further details of which are provided below. Also, in one embodiment of an expression apparatus 201 (Fig. 7), an outer shell 208 has a portion 208c (Figs. 9, 10) that is configured to extend over or near an axillary part 4b (Fig. 10) of the breast 4, as shown in Fig. 10. An inner surface 208d (Fig. 9) of the outer shell 208 is generally concave, and may be convex on portion 208c to conform to the contour of the axillary part 4b (Fig. 10) of the breast 4.

The expression apparatus 1 may include an outer cover 7 that may cover the outer shell 8 and/or the compression unit The outer cover 7 may function to protect the outer shell and/or the compression unit and may also provide a desired visual appearance. The expression apparatus 1 may also include an inner liner 7a to cover the pads 14 coupled to the outer shell 8. The inner liner 7a may provide a comfort barrier between the breast 4 and the pads 14. The outer cover 7 and/or inner liner 7a may be removable from the expression apparatus 1. The outer cover 7 and/or the inner liner 7a may be made from one or more of polyester, cotton, spandex, silk, or other preferably comfortable-to-the-touch material. In one embodiment, the outer cover 7 and the inner liner 7a are coupled together to form an encasement for the expression apparatus 1. While the outer shell 8 is shown as being a generally solid structure, in at least one embodiment, the outer shell 8 may be have openings and may take the form of a frame or cage-like structure, which may make the shell 8 lighter and more comfortable for the user to wear.

In at least one embodiment, the breastshield 6 may be incorporated into the expression apparatus 1. For example, a flange 6a (Fig. 2) of the breastshield 6 may be removably attachable to and detachable from the outer shell 8, outer cover 7, or inner liner 7a. For example, the flange 6a of the breastshield 6 may removably engage (e.g., adhesively, friction fit, interference fit, etc.) the outer shell 8, outer cover 7, or inner liner 7a to retain the breastshield 6 connected thereto when in use and permits the breastshield 6 to be detached from the outer shell 8, outer cover 7, or inner liner 7a, without damage to any of the breastshield 6, the outer shell 8, outer cover 7, and inner liner 7a.

The outer shell 8 and pads 14 may be made from one or more fabric materials. The fabric used for the outer shell 8 may be stiffer than the fabric used for the pads 14. Portions of the fabric(s) used may be reinforced or stiffened, such as with a wire, similar to an underwire of a bra. Such a reinforcement or stiffener may be made of at least one of metal, plastic, or resin, for example. Further, the outer shell 8 may be formed as a fabric covered cage or mesh, which may improve comfort for the user, as mentioned above.

The outer shell 8 has a deck 8a extending generally horizontally from a side of the outer shell 8. In a case where the outer shell 8 is made of fabric, as discussed above, the deck 8a may be formed from stiff fabric and may be reinforced with a reinforcement member or stiffener (e.g., wire), as noted above. The deck 8a is configured for mounting and supporting a drive unit 10, further details of which are provided below. For example, the drive unit 10 may be adhesively or mechanically attached to the deck 8a. The outer shell 8 also defines a circumferential through-slot 8b proximate the deck 8a which is configured to route a cable 12, which is driven by the drive unit 10. As will be described in greater detail hereinafter, the drive unit 10 and the cable 12 operate to cyclically compress and allow decompression of the pads 14 against the breast 4 to manipulate the tissue of the breast 4. The drive unit 10 and the cable 12 may thus be considered an embodiment of what is hereinafter termed a "compression unit" Other embodiments of a compression unit are possible. For example, in one embodiment a geared belt or notched strap may be used in place of the cable 12 and such a geared belt or notched strap may be moved by a releasable ratcheting arrangement to tighten the belt or strap and allow the belt or strap to be loosened.

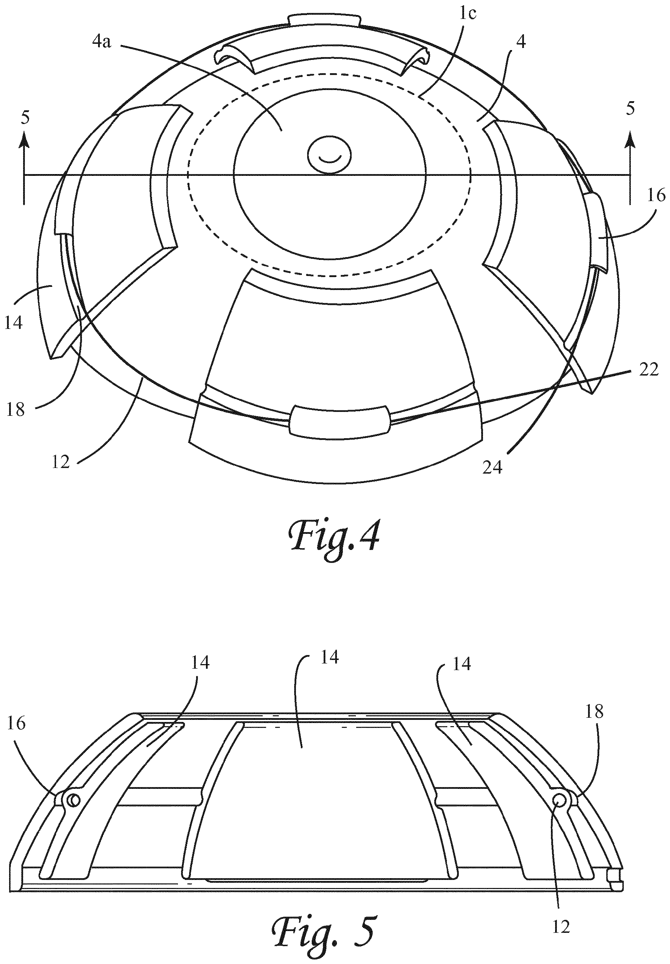

As shown in Fig. 3, the outer shell 8 partially covers a plurality of the pads 14. Fig. 4 shows the pads 14 with the outer shell 8 omitted for clarity. The pads 14 are spaced circumferentially from one another around the central opening 1c. The pads 14 may be made of flexible material, such as polyurethane or silicone. The pads 14 may have a Shore hardness of 10A to 90 D. The pads 14 are configured to be radially displaced inwardly for a certain period of time by action of the drive unit 10, which moves the pads 14 toward and against the breast 4, compressing the tissue of the breast 4. When the action of the drive unit 10 reverses, the compression ends and the pads 14 can radially expand due to radially outward pressure on the pads 14 applied by the breast 4. This compression/decompression cycle can repeat by operation of the drive unit 10. The parameters of that cycle may be adjusted, start, or stopped by the controller 2, which is communicatively coupled to the drive unit 10, either via a wired connection 2c or a wireless connection (e.g. infra-red, Wi-Fi, Bluetooth, etc.).

In one embodiment at least one or more of the pads 14 may have a heating element (not shown) to warm the breast 4 during operation of the expression apparatus 1. Also, optionally, on an inner side of the pads 14 (e.g., side facing the breast 4), the pads 14 may have one or more padding layers (not shown), such as to improve user comfort and fit In one embodiment, the pads 14 may contain a gel-like material to facilitate heat dispersion. The gel-like material may include silicone.

As shown in Figs. 4 and 5, each pad 14 defines an enclosed cable channel 16 and a groove 18 that extend circumferentially on an outer side of the pad 14. The cable channels 16 and grooves 18 are configured to route the cable 12 around the plurality of pads 14, thereby coupling the pads 14 together. The cable 12 crosses over itself (preferably without touching) between two of the pads 14, as shown in Figs. 3 and 4. While only one cable channel and groove are shown for each pad 14, each pad 14 may have more than one cable channel and groove to accommodate more than one cable, as will be described in greater detail below.

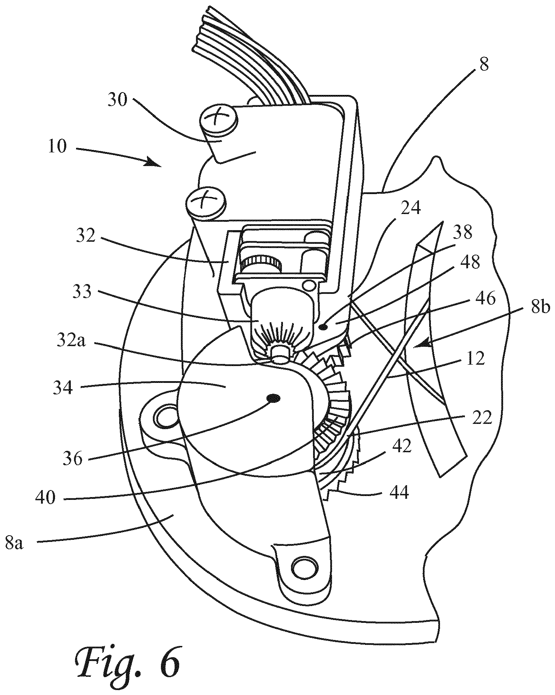

As shown in Fig. 6, the cable 12 extends through the circumferential slot 8b in the outer shell 8 and crosses over itself with a first end 22 and a second end 24 extending to the drive unit 10. In the embodiment of the drive unit 10 shown in Fig. 6, the drive unit 10 includes an electric motor 30, a gearbox 32 driven by the motor 30, and a transmission system 34 driven by the motor 30 and connected to the first and second ends 22 and 24 of the cable 12. The motor 30 may be an AC or DC electric motor powered by a power supply included with the expression apparatus 1, such as a battery, or by an external power supply, such as a power supply (e.g., battery) located in the controller 2 and supplied via cord 2c (Fig. 1A). Also, the expression apparatus 1 or the controller 2 may have a power connector for receiving power from an electrical outlet, such as an AC outlet.

The motor 30 may be coupled to the gearbox 32 by one or more gears as is known in the art to drive a driving bevel gear 33 on an output shaft 32a of the gearbox 32. The transmission system 34 includes a first vertical shaft 36 and a second vertical shaft 38 that are driven by the driving bevel gear 33. The first shaft 36 rotates about a first vertical axis along a longitudinal length of the first shaft 36. A driven bevel gear 40 is secured to an upper end of the first shaft 36. The driven bevel gear 40 meshes with and is configured to be driven by the driving bevel gear 33 to rotate the first shaft 36 about the first axis. The first shaft 36 passes through a center of a first pulley 42, which is secured to the first shaft 36 to rotate with the first shaft 36 about the first axis. The first pulley 42 is secured to the first end 22 of the cable 12. A driving gear 44 is secured to a lower end of the first shaft 36 for rotation therewith.

The second shaft 38 extends parallel to the first shaft 36 and the second shaft 38 is configured to rotate about a second vertical axis along a longitudinal length of the second shaft 38. A driven gear 46 is secured to a lower end of the second shaft 38 and is configured to rotate about the second axis with the second shaft 38. The driven gear 46 is enmeshed with the driving gear 44, which drives the driven gear 46 and rotates the second shaft 38 when the first shaft 36 rotates. However, the direction of rotation of the first and second shafts 36 and 38 is opposite (i.e., clockwise and counterclockwise or vice versa depending on the direction of movement of the motor). The second shaft 38 extends through a center of a second pulley 48, which is secured to the second shaft 38 for rotation with the second shaft 38 about the second axis. The second end 24 of the cable 12 is secured to the second pulley 48. An upper end of the second shaft 38 is preferably supported by a housing of the motor 30. As a result of the gearing of the transmission system 34, when the motor 30 operates so as to rotate the driving bevel gear 33 in a clockwise direction, the first shaft 36 and first pulley 42 are rotated clockwise, and the second shaft 38 and the second pulley 48 are rotated counter-clockwise so that a certain length of the cable 12 from its respective ends 22 and 24 will be taken up by the respective pulleys, which will draw the pads 14 radially inward relative to opening 1c to cause compression of the breast 4. When the motor 30 reverses direction, the cable 12 slackens (relaxes) allowing the compressed breast 4 to relax and decompress. As the breast 4 decompresses, the tissue of the breast 4 pushes the pads 14 radially outwardly, while at least some of the length of the cable 12 taken up during compression is withdrawn from the pulleys 42 and 48.

Each tensioning and relaxation of the cable 12 which results in compression and permits decompression of the breast may be considered a cycle, which is preferably repeated to effect a breast tissue manipulation, which may cause breast milk expression. Parameters of the cycle(s) can be controlled by the controller 2. For example, the controller 2 may be configured to control the duration of compression and the duration of decompression, which may be the same or different Also, the controller 2 may be configured to control the tension of the cable 12 during compression (which can control the pressure applied to the breast). The controller 2 has switches and buttons 2a and 2b, which may control the operating parameters as well as to turn the expression apparatuses on and off.

The controller 2 may be configured to operate based on set operating parameters or a real-time operating state of a breast pump apparatus that may be used simultaneously with the system 100 by the user, as discussed above. For example, the user of the system 100 may use the controller 2 to a select a manipulation speed or pressure, such as with the buttons 2a, 2b, based on the operating state of the breast pump to achieve a desired breast pumping result Specifically, the user may select a certain compression and decompression duration and/or compression pressure based on a breast pumping speed and/or breast pumping pressure setting of the breast pump apparatus to achieve, for example, optimal milk flow rate or a desired level of comfort

Alternatively or additionally, the controller 2 may be communicatively coupled (via wired or wireless connection) to a computing device 13 (Fig. 1), such as personal computer, smart phone, smart watch, or tablet computer, which can set and control the operation of the expression apparatus(s) 1. The computing device 13 may be coupled to a graphical display device (e.g., a screen). The computing device 13 may execute software (e.g. an application, a.k.a., an "app") that causes the display device to graphically display an operational interface to a user. The operational interface may be used to configure and/or control the expression apparatuses 1. For example, the operational interface may include virtual controls (e.g., on-screen buttons) that may replicate the physical buttons 2a, 2b of controller 2, and may provide information to the user about an operating status of the system 100. By way of example, and not limitation, such information may include current operating setting(s), elapsed time of use, heater temperature level, and breast skin temperature. The provided information may be saved locally or remotely for retrieval and/or analysis.

In one embodiment, the controller 2 may be communicatively coupled (via a wired or wireless connection interface) to a breast pump apparatus to automatically control the operation of the system 100 based upon the operation of the breast pump apparatus. For example, the controller 2 may automatically synchronize the operation of one or both expression apparatuses 1 of the system 100 with the breast pump apparatus based on feedback received directly from the breast pump apparatus or from a user of the breast pump apparatus. In one embodiment, a closed-loop feedback system may include a sensor that monitors a rate of milk production of the breastfeeding apparatus. The rate of milk production can be used as an input to the controller 2 as a basis to adjust operating parameters and settings of the system 100 so as to regulate the rate of milk production.

An example of an embodiment of an expression apparatus 201 having multiple cable channels, grooves, and cables is shown in Figs. 7 to 9. The expression apparatus 201 has the same function as the expression apparatus 1, but differs in structure in that the expression apparatus 201 includes a plurality of cable channels, grooves, and cables, further details of which are provided below. In the embodiment of the expression apparatus 201, elements corresponding to expression apparatus 1 are incremented by "200". Thus, as shown in Fig. 7, the expression apparatus 201 has an outer shell 208 with a deck 208a that supports a drive unit 210.

As shown in Fig. 8, the expression apparatus 201 has a plurality of pads 214 on an inner side of the outer shell 208. The pads 214 have a plurality of channels 216a and 216b through which corresponding cables 212a and 212b extend. For each pad 214, channel 216a is spaced from channel 216b, and channel 216a is located closer to opening 201c in the expression apparatus 201 than channel 216b.

The outer shell 208a defines openings 208b' and 208b" through which corresponding cables 212a and 212b extend, as shown in Figs. 7, 7A, and 9. As shown in greatest detail in Fig. 7A, cable 212a has ends 222a and 224a that are routed to a transmission system 234 of the drive unit 210. The drive unit 210 includes an electric motor 230, a gearbox 232 driven by the motor 230, and the transmission system 234 driven by the motor 230. End 222a is routed to a pulley 242a on shaft 236 of the transmission system 234 and end 224a is routed to a pulley 248a on shaft 238 of the transmission system 234. The other cable 212b has ends 222b and 224b that are routed to the transmission system 234 of the drive unit 210. Specifically, end 222b is routed to a pulley 242b on shaft 236 and end 224b is routed to a pulley 248b on shaft 238. The shafts 236 and 238 correspond to shafts 36 and 38 of drive unit 10 and shafts 236 and 238 are coupled together and driven by a geared arrangement in the same manner as shafts 36 and 38. Thus, a detailed description of the drive unit 210 and its operation in driving shafts 236 and 238 with the motor 230 and gearbox 232 is omitted for brevity. As noted above, the operation of the expression apparatus 201 is the same as for expression apparatus 1. When the drive unit 210 is operating in one direction, it tensions both of the cables 212a and 212b around the pads 214 to compress the breast When the drive unit 210 reverses direction, cables 212a and 212b loosen (relax) and permit the breast to decompress.

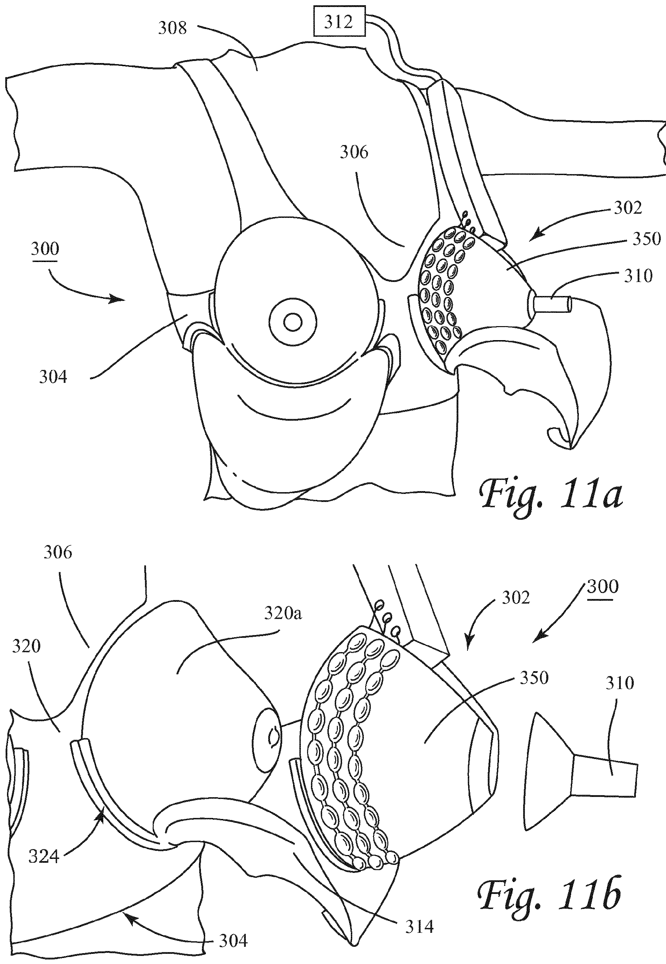

Figs. 11a and 11b show another embodiment of a breast milk expression system 300. The system 300 includes a breast milk expression apparatus 302 and a nursing-style bra 304 which is removably connected to the breast milk expression apparatus 302. The breast milk expression apparatus 302 includes a compression pad 350 that is configured to squeeze a breast of a user 308 to perform breast milk expression. The bra 304 is configured to hold the breast milk expression apparatus 302, and specifically the compression pad 350, in fixed relation with respect to the user's breast 306. As will be appreciated, a breastshield 310 can be used with the system 300 and may be located between an inner cup 320a (e.g., inner surface of inner cup 320a) of the bra 304 and compressive pad 350. Preferably, the bra 304 is connected to the breast milk expression apparatus 302 and the system 300 is used like a bra, but also facilitates hands-free operation of one or two (if present) of the expression apparatuses 302. Each expression apparatus 302 (only one expression apparatus 302 is shown in Figs. 11a and 11b) is configured to manipulate the tissue of the corresponding breast 306 based on a control input from a controller 312 (Fig. 11a), as described in greater detail below. The milk expression system 300 can be used before, during, and/or after breast pumping or nursing to facilitate lactation and improve milk production and flow. Further details of the breast milk expression system 300 and its operation will now be described.

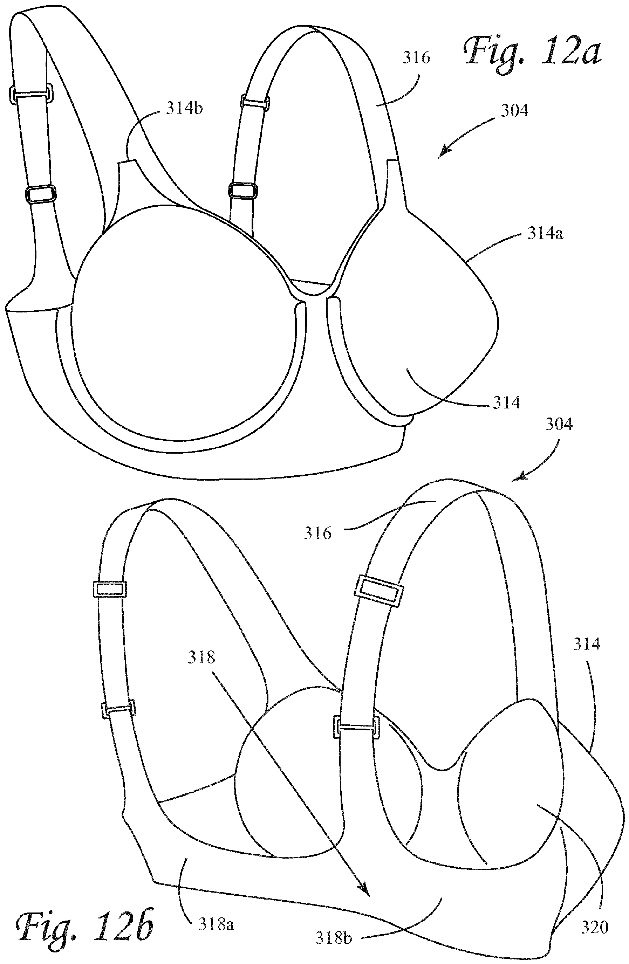

Figs. 12a to 12d show details of an embodiment of the bra 304 of the system. In the embodiment, the bra 304 is formed as a nursing-style bra with an outer layer 314 having folding cups 314a, an inner layer 320 (Fig. 12b) configured to be in contact with the breast 306, and adjustable shoulder straps 316. The bra 304 may come in various sizes based on the breast size of the user 308. The straps 316 are adjustable in length and are configured to support one or two expression apparatuses 302 over respective breasts 306 without the user 308 holding the expression apparatuses 302 in place, regardless of whether the expression apparatuses 302 are turned on or off.

As shown in Fig. 12b, at the back of the bra 304, the straps 316 connect to a corresponding back strap 318, which may be adjustable in length. The back strap 318 may be continuous or may be formed of segments. For example, in one embodiment, the back strap 318 is formed of a left segment 318a and a right segment 318b that are selectively connected in the middle of the strap with a suitable connector (not shown), which may permit adjustment of the length of the back strap 318.

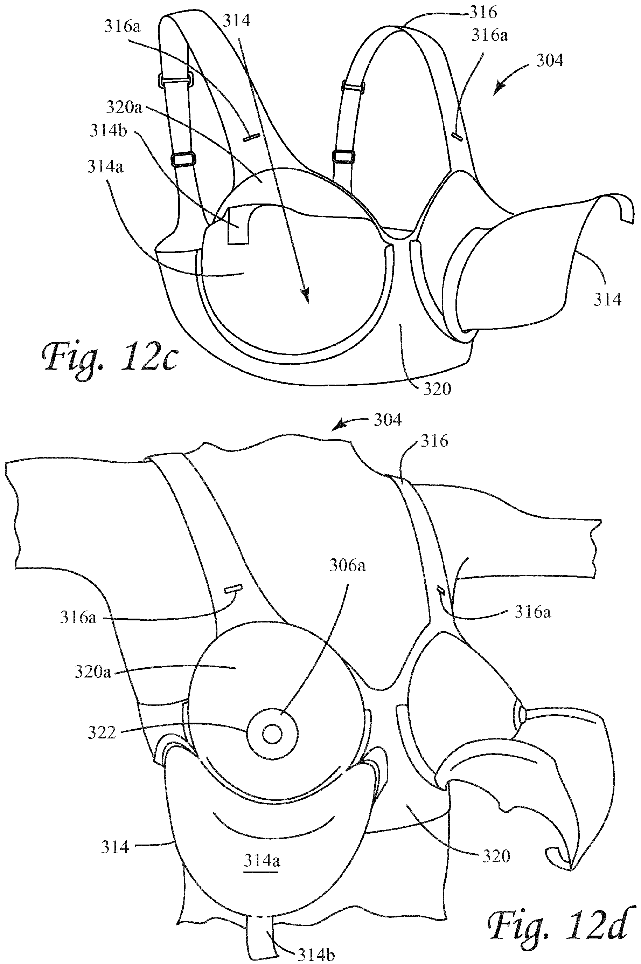

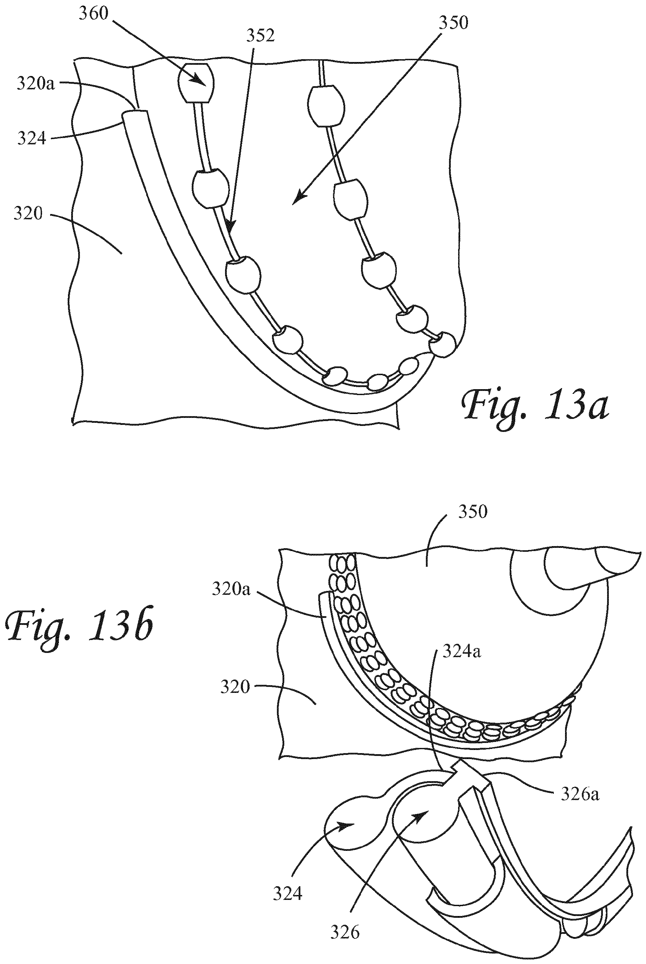

As shown in Figs. 12c and 12d, the cups 314a are configured as portions of the outer layer 314 of the bra 304 and can fold down independently of one another to expose respective inner cups 320a formed in the inner layer 320 of the bra 304. Each inner cup 320a, which may also be independently foldable, defines a hole 322 (Fig. 12d) located around a nipple area 306a (Fig. 12d) of the breast 306. Each inner cup 320a may have a removable connector 320b (Fig. 13b) (e.g., a snap connector, hook, or loop) at its upper end and a connector (e.g., stitching) at its bottom. The upper connector 320b may be removably connected to a connector 316b (Fig. 13b) (e.g., a snap connector, hook, or loop) on the strap 316. Such an arrangement may permit a user to disconnect the inner cup 320a at the upper connector and fold the inner cup 320a about the lower connector to permit the user to access the breast tissue, and specifically the nipple area 306a, for positioning the breastshield 310 thereon. The hole 322 is configured and sized so that a tubular part of the breastshield 310 (Fig. 11b) can extend through the hole 322 while a conical flange part of the breastshield 310 can be retained in engagement with the nipple area 306a between the inner cup 320a of the inner layer 320 and the nipple area 306a (i.e., in contact with the nipple area 306a).

Also, as shown in Figs. 12c and 12d, each outer cup 314a may have a tab 314b that removably connects to a portion 316a of the strap 316. The connection between the tab 314b and the portion 316a may be a hook and loop connection, snap fit, and the like which allow the outer cup 314a to remain in place covering the inner cup 320a and the breast 306 or allow the cup 314a to fold away when so desired.

The expression apparatus 302 is configured to removably connect to the bra 304. One embodiment of a connection is shown in Figs. 13a and 13b. As shown in Figs. 13a and 13b, an attachment channel 324 is connected to (e.g., sewn to) the inner layer 320 and extends about the bottom at the base of the inner cup 320a of the inner layer 320, as shown with momentary reference to Fig. 11b. The attachment channel 324 is configured to mate with a corresponding channel 326 (Fig. 13b) affixed to the expression apparatus 302. In the embodiment shown in Fig. 13b, the channel 324 has a female circular profile with a slot 324a and the channel 326 has a male, solid circular profile with a "T" shaped portion 326a extending through the slot 324a. The "T" shaped portion 326a is fixed to the pad 350, e.g., sewn connection or adhesive.

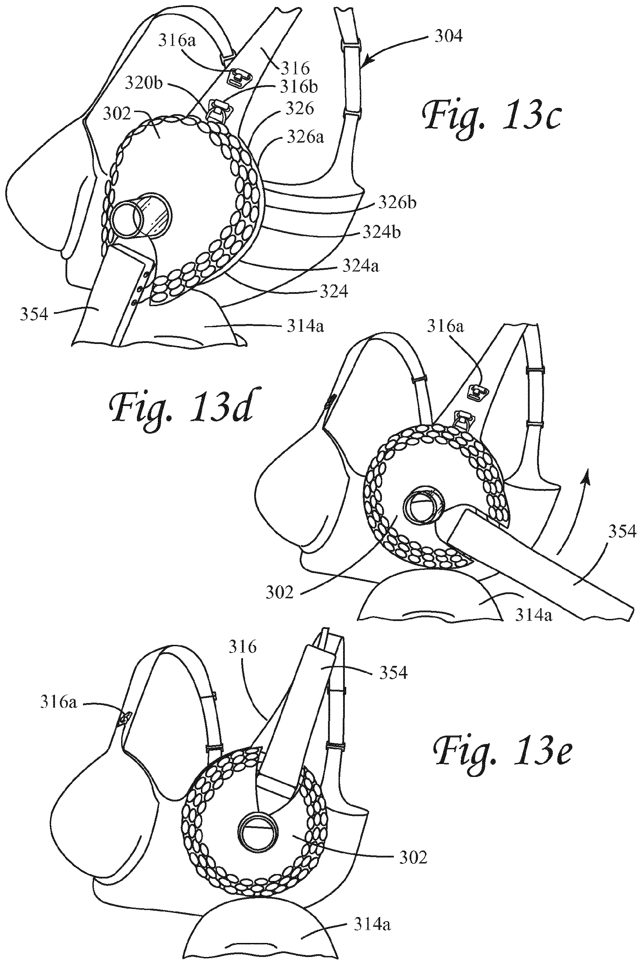

Figs. 13c to 13e show a sequence for attaching the expression apparatus 302 to the bra 304 using the attachment channel 324 and the channel 326. As shown in Fig. 13c, the attachment channel 324 and the channel 326 are substantially semicircular and a user can position the expression apparatus 302 such that the channel 326 is aligned above the attachment channel 324 so that the attachment channel 324 and the channel 326 form a substantially circular pattern. Specifically, in Fig. 13c, ends 324b of slot 324a are aligned with respective ends 326b of the T-shaped portion 326a on both left and right sides of the expression apparatus 302, though only the right side is shown in Fig. 13c. Also, in Fig. 13c, a routing module 354 (described in greater detail below), extends downwardly from the expression apparatus 302. With the ends 324b and 326b aligned, the expression apparatus 302 can be rotated in the direction of the arrow (e.g. counter-clockwise) of Fig. 13d to further engage the attachment channel 324 and the channel 326. The expression apparatus 302 is rotated until the channel 326 is received and extends fully within the attachment channel 324 and the routing module 354 extends substantially in alignment with shoulder strap 316 (as seen in Fig. 13e), whereupon the routing module 354 may be removably attached to the strap 316 with suitable connectors, which may attach to connection 316a (Figs. 13d and 13e), for example.

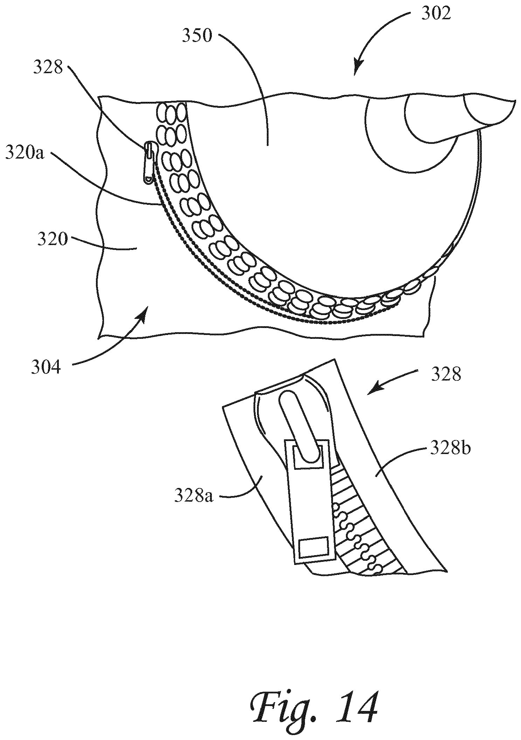

A second embodiment of a connection between the expression apparatus 302 and the bra 304 is shown in Fig. 14, which shows a zipper 328 used as the connection. Specifically, one half 328a of the zipper 328 extends along a lower half of the inner cup 320a of the inner layer 320 and another half 328b is connected around (e.g. sewn to) a lower portion of the expression apparatus 302. The halves 328a and 328b are configured to mesh or zip together to connect the expression apparatus 302 to the bra 304 and the halves 328a and 328b are configured to unmesh or unzip to disconnect the expression apparatus 302 from the bra 304. In one aspect, other means of attachment such as clips, or hooks and loops (e.g., Velcro) may be utilized.

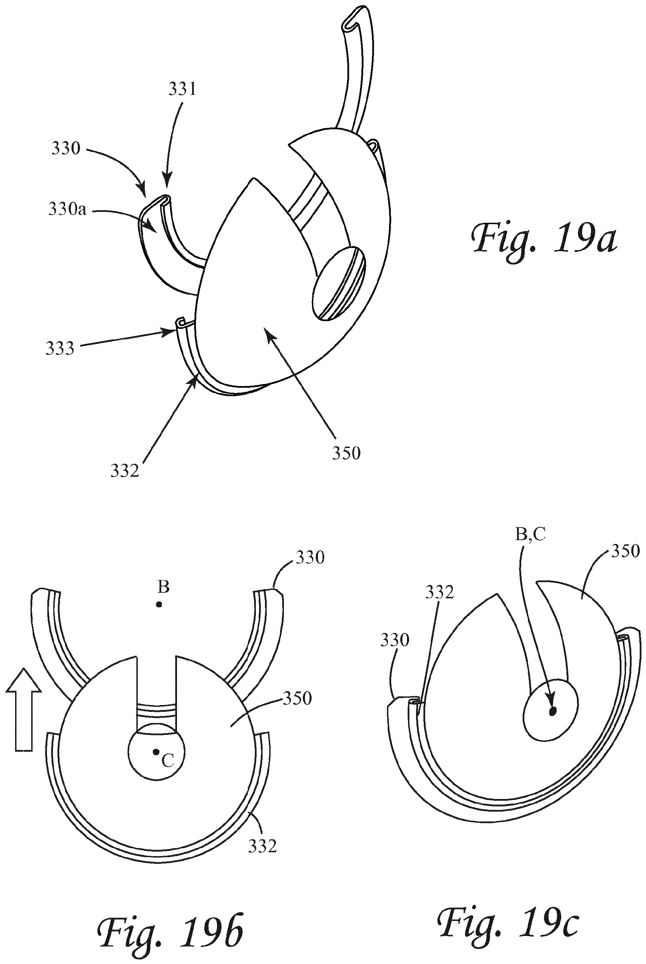

A third embodiment of a connection between the expression apparatus 302 and the bra 304 is shown in Figs. 19a to 19c, which shows a first hook 330 having a female channel 331 and a second hook 332 having a male channel 333. The first hook 330 has a flange 330a which can be securely fastened to the inner layer 320 under the inner cup 320a. The second hook 332 extends around the lower half of the compression pad 350. The first hook 330 and the second hook 332 extend circumferentially about respective axes B and C, as shown in Fig. 19b. The female channel 331 and the male channel 333 are configured to slide into engagement as shown in Fig. 19b in the direction of the arrow so that the axes B and C come into alignment when the first hook 330 and the second hook 332 are fully connected, as shown in Fig. 19c. A diameter of the second hook 332 is slightly smaller than a diameter of the first hook 330 to permit the second hook 332 to expand slightly and snap onto the first hook 330 when the second hook 332 is slid upwardly in the direction of the arrow shown in Fig. 19b. Although the routing module 354 in Figs. 13c to 13e is not shown in Figs. 19a to 19c for clarity of illustration, the module 354 may be present and connected to pad 350 in Figs. 19a to 19c and the module 354 may be connected to strap 316 of bra 304. Such connection of the module 354 to the strap 316 may be useful for providing additional support to the pad 350 to prevent disconnection of the second hook 332 from the first hook 330 due to the weight of the pad 350.

It will be appreciated that the bra 304 may be connected to structures other than the expression apparatus 302. For example, heating pads and cooling pads can be configured to have connections like those shown in Figs. 13a, 13b, and 14 to connect to the bra 304 to apply, respectively, heating or cooling to the breast 306. Also, the other structures may include medicated pads, which when connected to the bra, may be configured to deliver medication to the breast 306.

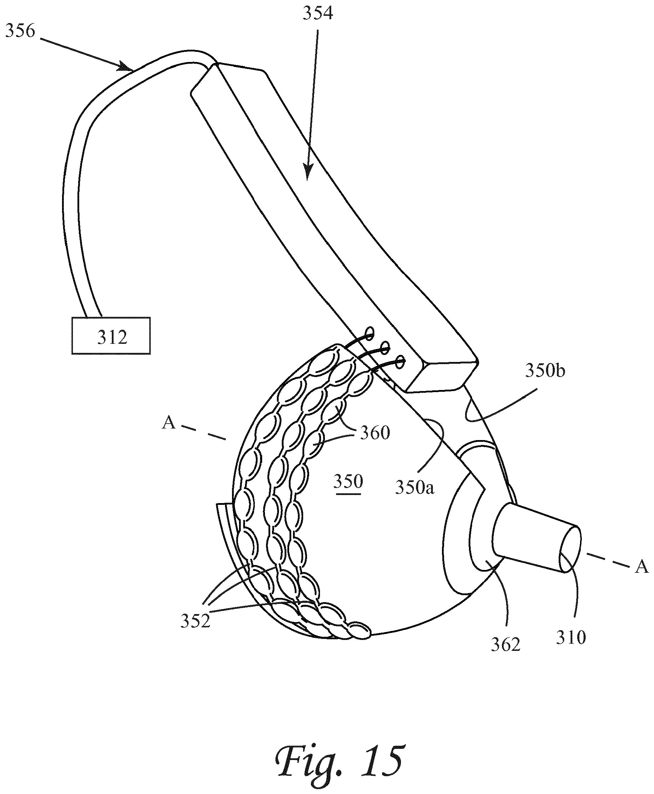

Fig. 15 shows additional details of the expression apparatus 302. The expression apparatus 302 includes a compression pad 350 having a plurality of strings 352 arranged concentrically with axis A-A. For purposes herein, the term "string" is to be understood broadly to include monofilaments, multifilaments, twisted filaments, etc., which may be comprised of any of several materials such as metal, polymers which are substantially inelastic in tension, or other suitable materials, or combinations thereof. The expression apparatus 302 also includes a routing module 354 and a gearbox 312 (shown schematically) coupled to the strings 352. In one embodiment, each respective string 352 has one end that terminates in the module 354 and an opposite end that terminates in the gearbox 312. In another embodiment, each respective string 352 has two ends that terminate in the gearbox 312. In the embodiment shown in Fig. 15, the strings 352 are routed between the module 354 and the gearbox 312 by a tether 356. In another embodiment, instead of a single tether 356 routing all of the strings 352, a plurality of tethers may be used to individually route each string between the module 354 and the gearbox 312. It will be appreciated that in one embodiment, the gearbox 312 is integrated with the module 354 eliminating the tether 356. The tether 356 is preferably sufficiently long so as to permit the user 308 to dispose the gearbox 312 below the user's breast 306, and, preferably, proximate to a waist of the user 308. As will be described in greater detail below, the gearbox 312 is configured to pull on the strings 352 to radially compress the compression pad 350 and the breast 306 (e.g., via the inner cup 320a of the inner layer 320).

Each string 352 is routed through a corresponding plurality of circumferentially spaced beads 360, which are secured to the compression pad 350. Each string 352 is configured to slide freely through an opening defined in each bead 360. The beads 360 may have a flat outer surface, as illustrated in Fig. 15, or they may have other outer shapes, such as cylindrical and round (e.g., spherical). The beads 360 may be secured to the compression pad 350 with adhesive (e.g., glue) or with mechanical fasteners (e.g., by sewing) and may be secured either on the outer surface of the pad 350 or within channels (not shown) recessed into the outer surface of the pad 350. As an alternative to the beads 360, the strings 352 may be routed through tubular channels secured to the pad 350. Such tubular channels may be secured to the outer surface of the pad 350, or may be secured in recessed channels formed in the outer surface of the pad 350. Such tubular channels may be formed by sewing material (e.g., fabric) into a tube and connecting the tube to the pad 350. Also, the aforementioned tubular channels may be a single continuous channel or may be a plurality of tubular channels circumferentially spaced about axis A-A approximating the arrangements (e.g., number and spacing) of the beads 360 shown in Fig. 15. The strings 352 are axially spaced from one another along axis A-A.

The number and longitudinal spacing of the strings 352 may be determined based on at least one of the size of the breast 306 and the timing of a compression sequence, further details of which are described below. Also, the number and circumferential spacing of the beads 360 along each string 352 may be determined based on atleastthe size of the breast 306, with the either more beads being used at the same spacing for an expression apparatus accommodating a relatively larger breast, or the same number of beads being spaced further apart, or both.

The compression pad 350 may be made from fabric, polymer, or any flexible material. The compression pad 350 defines a hole 362 at a distal end corresponding to the nipple area 306a (Fig. 12d) and aligned with the hole 322 (Fig. 12d) in inner cup 320a (Fig. 12d). The hole 362 is configured to receive therethrough a portion of the breast shield 310, as shown in Fig.15.

The compression pad 350 has an outer surface that is substantially tapered (e.g., frustoconical or convex) along axis A-A and which is substantially (at least 75%) continuous about axis A-A. In the embodiment shown in Fig. 15, the pad 350 is discontinuous about axis A-A and extends from one end 350a to an opposite end 350b. The module 354 is disposed in spaced relation to and between the ends 350a and 350b of the compression pad 350 so that the module 354 directly contacts the inner cup 320a (Fig. 11b) of the inner layer 320 (Fig. 11b). It will be appreciated, however, that the pad 350, in at least one other embodiment, may be circumferentially continuous and extend 360 degrees around axis A-A.

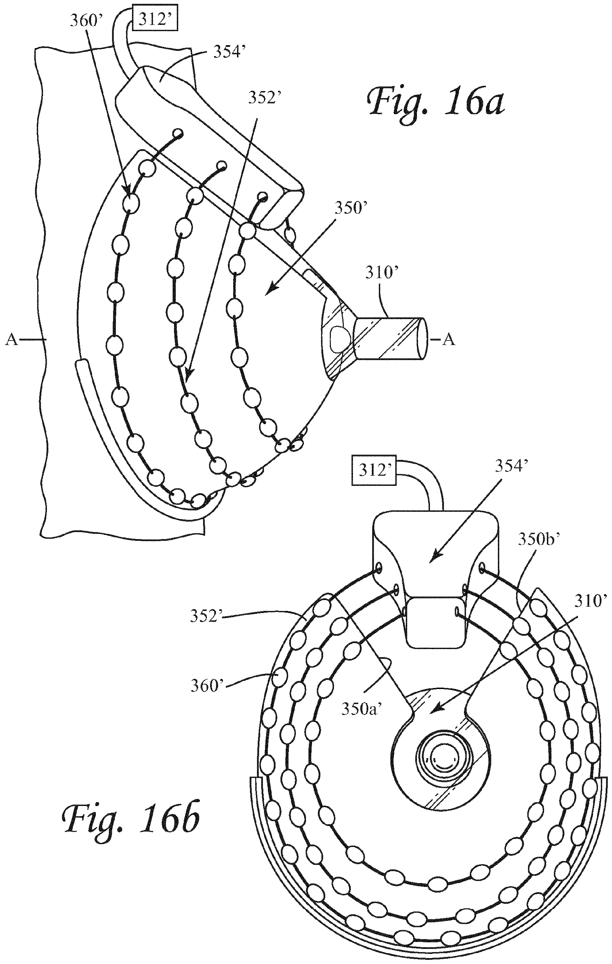

Figs. 16a and 16b show another embodiment of the expression apparatus 302' which is the same as expression apparatus 302 with the exception of routing module 354', which is shorter than module 354 and has curved sides instead of straight sides of slimmer module 354. In Figs. 16a and 16, elements of the expression apparatus 302' that correspond to elements of the expression apparatus 302 are appended with an apostrophe (').

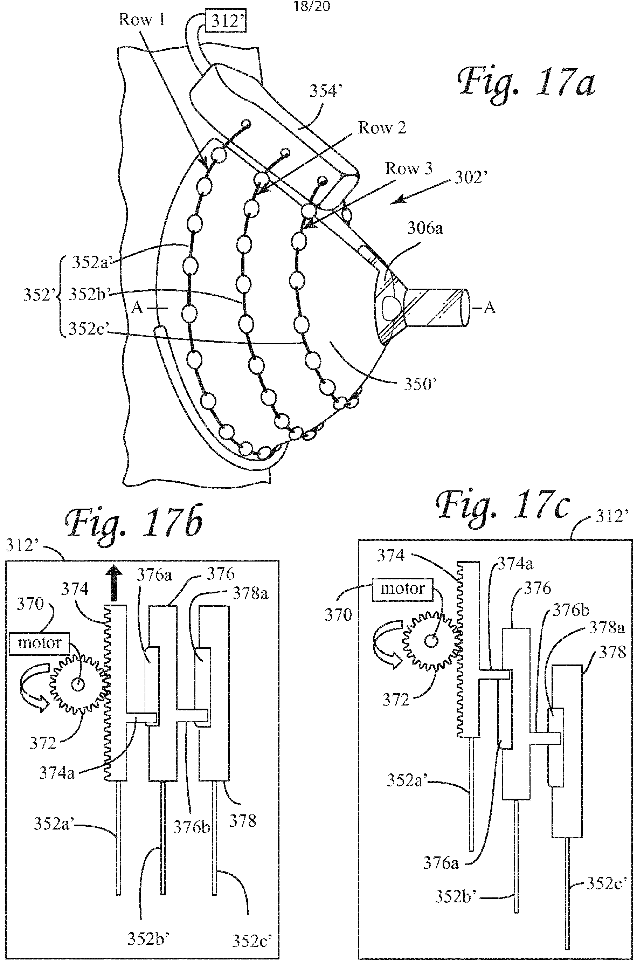

Figs. 17a to 17c illustrate further details of the expression apparatus 302'. In Figs. 17a to 17c, the plurality of strings 352' includes inner string 352a' (in relation to the torso of the user), middle string 352b', and outer string 352c' (being closest to the nipple area 306a along axis A-A). The gearbox 312' is configured to pull on respective ends of the strings 352a', 352b', and 352c' to concentrically tighten the respective strings around the pad 350', and, therefore, around the breast 306 to radially compress the breast 306. Specifically, with respect to the embodiment of the expression apparatus 302' shown in Figs. 16a, 16b, and 17a, when one or more of the strings 352a', 352b', and 352c' are tightened by the gearbox 312' they compress the sides and underside of the breast 306 through the compression pad 350' and inner cup 320a (Fig. 11b) of the inner layer 320 (Fig. 11b), while the module 354' compresses the top of the breast 306 through the inner cup 320a (Fig. 11b). The module 354' may be shaped in various ways to cover more or less of the inner cup 320a (Fig. 11b).

In one embodiment, the gearbox 312' is configured to tighten the strings 352a', 352b', and 352c' in a compression sequence, one of which is described below with reference to Figs. 17b and 17c. The gearbox 312' includes a motor 370 and a pinion gear 372 driven by the motor 370. The motor 370 may be powered by an electrical power source, such as an AC or DC power source, which may be internal or external from the gearbox 312'. In the gearbox 312' the end of inner string 352a' is connected to a rack 374 that is enmeshed with the pinion gear 372. The rack 374 and the inner string 352a' translate parallel to the length of the inner string 352a' (upwardly in Figs. 17b and 17c) in the gearbox 312' when the pinion gear 372 rotates counter-clockwise. The end of the middle string 352b' is connected to a middle pull member 376 and the end of the outer string 352c' is connected to a pull member 378. The middle pull member 376 and the outer pull member 378 have respective elongated notches 376a and 378a defined along the length of the respective pull member. An engagement tab 374a extends to the right from the rack 374 into the elongated notch 376a of the middle pull member 376, and another engagement tab 376b extends from middle pull member 376 into the elongated notch 378a of the outer pull member 378.

The relative positions of the strings 352a', 352b', and 352c' shown in Fig. 17b is a neutral position in which all of the strings 352a', 352b', and 352c' are not in tension, corresponding to a position in which the breast 306 is not being compressed by the expression apparatus 302'. Whenever any of the rack 374, middle pull member 376, or pull member 378 is displaced (upward in Figs. 17b and 17c) away from its respective neutral position, such displacement causes radial displacement of the compression pad 350 and compression of the breast 306 of the user 308. Owing to the arrangement of the notches 376a and 378a and the engagement members 374a and 376b, the timing of and sequence of moving each string 352a', 352b', and 352c' can be controlled during each upward stroke of the rack 374, corresponding to a compression phase of a compression-expansion cycle.

The operation of the gearbox 312' may be understood with reference to Fig. 17b and Fig. 17c. As the pinion gear 372 rotates counterclockwise in Fig. 17b, the rack 374 and engagement tab 374a translate upward until the engagement tab 374a engages the middle pull member 376 at the top of the elongated notch 376a, as shown in the position in Fig. 17c. Thereafter, continued counterclockwise rotation of the pinion gear 372 will cause both the rack 374 and the middle pull member 376 to move upwardly together until the engagement member 376b engages the outer pull member 378 at the top of the elongated notch 378a. Thereafter, continued counterclockwise rotation of the pinion gear 372 will cause the rack 374, middle pull member 376, and outer pull member 378 to move upward together until the rack 374 stops moving, thereby ending the upward compression stroke of the rack 374. Thereafter, the motor 370 can be temporarily turned off to begin an expansion stroke of the rack 374 and an expansion phase of the compression-expansion cycle upon which tension in the wires 352a', 352b' and 352c' is removed, allowing outward expansion forces exerted from the breast 306 to urge the strings 352a', 352b', and 352c' back to their neutral positions shown in Fig. 17b, completing the compression-expansion cycle. The compression-expansion cycle can be repeated multiple times to facilitate breast milk expression. Thus, based on the compression sequence, the inner string 352a' will compress the breast 306 first, followed by the middle string 352b', and lastly the outer string 352c', such that the breast 306 will be compressed progressively in an outward direction (longitudinal along axis A-A) from the base of the breast 306 toward the nipple area 306a (Figs. 12d and 17a). Also, it will be appreciated that during each compression stroke of the rack 374, the string 352a' will be in tension for the longest period of time, followed by the middle string 352b', followed by the outer string 352c'.

While the gearbox 312' shown in Figs. 17b and 17c is linear, the gearbox design may be circular or elliptical. Also, although not shown in Figs. 17b and 17c, the gearbox 312' may be contained in a control box along with other electrical components for controlling the operation of the motor 370.

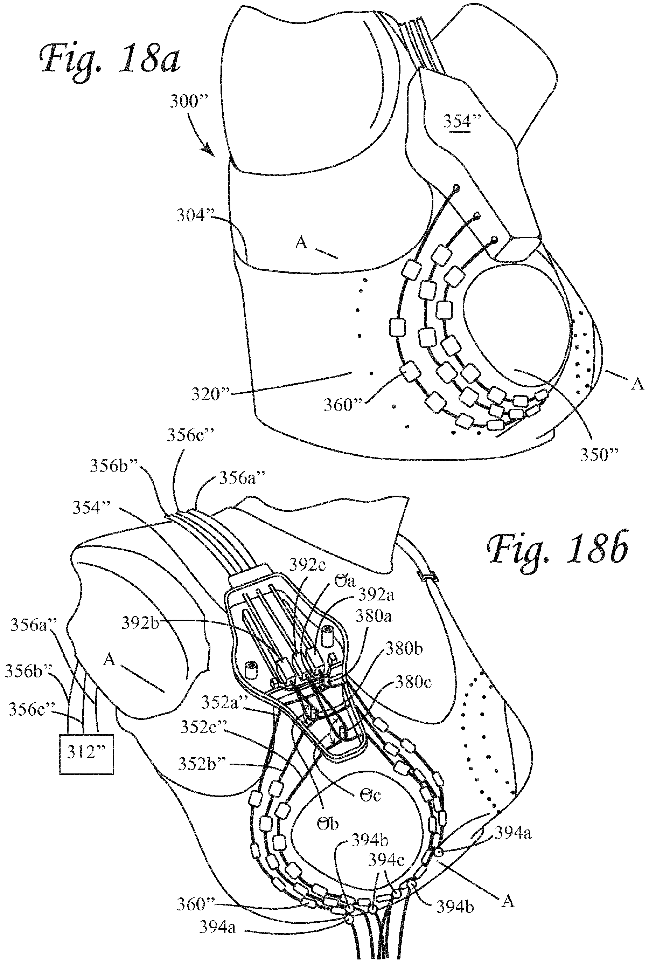

Figs. 18a and 18b show an alternative embodiment of the system 300, and denoted 300". In Figs. 18a and 18b elements of system 300" corresponding to those of system 300 are appended with two apostrophes ("). One notable difference between system 300" and system 300 is that the compression pad 350" is integrally formed with an inner layer 320" of a bra 304" and is not readily removable therefrom. Although not shown in Figs. 18a and 18b, an outer layer may optionally be used to cover or enclose the compression pad(s) 350" and inner layer 320".

Fig. 18b shows another embodiment of a module 354" with an outer cover thereof (shown in Fig. 18a) removed to show a routing of three strings: string 352a", string 352b", and string 352c". The module 354" has three connection blocks 392a, 392b, and 392c, which are respectively connected to strings 352a", 352b", and 352c". Specifically, inside each connection block 392a, 392b, and 392c is a pin (not shown), around which respective strings 352a", 352b", and 352c" are looped. From connection block 392a, ends of string 352a" are routed around an upper pin 380a so that the string 352a" extends circumferentially around the compression pad 350". Ends of the string 352a" are formed into knots 392a adjacentto beads 360" through which the string 352a" extends. Preferably, the knots 394a are located diametrically opposite (or close to that location) the block 392a about the pad 350". From connection block 392b, ends of string 352b" are routed around an upper pin 380b so that the string 352b" extends circumferentially around the compression pad 350". Ends of the string 352b" are formed into knots 392b adjacent to beads 360" through which the string 352b" extends. Preferably, the knots 394b are located diametrically opposite (or close to that location) the block 392b about the pad 350". From connection block 392c, ends of string 352c" are routed around an upper pin 380c so that the string 352c" extends circumferentially around the compression pad 350". Ends of the string 352c" are formed into knots 392c adjacentto beads 360" through which the string 352c" extends. Preferably, the knots 394c are located diametrically opposite (or close to that location) the block 392c about the pad 350".

Also, the connection blocks 392a, 392b, and 392c are respectively connected to wires 356a", 356b", and 356c", which are also operably connected to gearbox 312". The blocks 392a, 392b, and 392c are operably moved up and down in the module 354" by movement of the cables 356a", 356b", and 356c", which is caused by operation of the gearbox 312". The blocks 392a, 392b, and 392c are operably moved by the cables 356a", 356b", and 356c" and gearbox 312" to move the respective strings 352a", 352b", and 352c" relative to the pad 350 to cause compression of the breast 306, as described in greater detail below.

Each string 352a", 352b", and 352c" passes from the compression pad 350" through the sides of the module 354" and extends to their respective pins 380a, 380b, and 380c, and each respective string is routed upward from each respective pin to their respective blocks 392a, 392b, and 392c. The pins 380a, 380b, and 380c are located within module 354" so that the strings 352a", 352b", and 352c" are turned through respective angles θₐ, θ_{b}, and θ_{c} so that the strings 352a", 352b", and 352c" are routed upwardly to the respective blocks 392a, 392b, and 392c. Thus, the pins 380a, 380b, and 380c can provide mechanical advantage for the gearbox 312" to pull the strings 352a", 352b", and 352c" so that when the strings are pulled, the tension in the strings will cause compression of the breast 306 to be radially directed substantially all the way around the breast 306. In one embodiment, the angles θa, θb, and θc may be in a range of 100º and 160º.

There have been described and illustrated herein several embodiments of a breast milk expression system and apparatus. While particular embodiments of the invention have been described, it is not intended that the invention be limited thereto. Thus, while particular materials have been disclosed, it will be appreciated that other suitable materials may be used as well. In addition, while particular types of driving units have been disclosed, it will be understood that other arrangements of driving units can be used, such as, by way of example, and not by way of limitation, a motorized worm drive, and an electro-magnetic solenoid. Further, while particular embodiments using two or three strings for applying compression have been shown, it will be appreciated that four or more strings may also be utilized. The number or strings may be related to cup size. Also, while a system having two expression apparatuses is preferred, it will be recognized that a single expression apparatus system may also be used. Moreover, while particular configurations have been disclosed in reference to the straps used to position the expression apparatuses, it will be appreciated that other configurations could be used as well. It will therefore be appreciated by those skilled in the art that yet other modifications could be made to the provided invention without deviating from its scope as claimed.

## Claims

1. A breast milk expression apparatus (302, 302') comprising:
a single compression pad (350, 350', 350") disposable about a breast of a user and configured for radial displacement relative to said breast, wherein the compression pad has a tapered outer surface and extends longitudinally from a larger diameter first end to a smaller diameter second end; and
a compression unit coupled to the compression pad (350, 350', 350"), wherein the compression unit comprises a plurality of cables (352, 352', 352") banded about and through the compression pad, and a drive unit (312, 312', 312") coupled to the plurality of cables, the drive unit configured to apply tension to the plurality of cables to cyclically displace the compression pad (350, 350', 350") radially inwardly to cause compression of the breast and, following compression of the breast, permitting the breast to expand and decompress;
wherein the plurality of cables include a first cable (352a', 352a") located at a first longitudinal position and a second cable (352b', 352b"; 352c', 352c") located at a second longitudinal position that is longitudinally spaced from the first longitudinal position and closer to the second end;
wherein each cable of the plurality of cables is coupled to and extends circumferentially around an outer surface of the compression pad (350, 350', 350"), and wherein each cable is configured to slide relative to the compression pad; and
wherein the drive unit (312, 312', 312") includes a motor (370) and a transmission element (372, 374, 376, 378) driven by the motor, the transmission element coupled to the plurality of cables and configured to sequentially tension the cables and **characterized in that** the transmission element is configured to sequentially tension the cables
such that tension to the first cable (352a') 352a") is applied for a longer duration than to the second cable (352b', 352b"; 352c', 352c").

2. The apparatus according to claim 1, wherein the compression pad (350, 350', 350") defines a circumferential channel through which the plurality of cables (352, 352', 252") extend, wherein the circumferential channel permits relative movement of the plurality of cables relative to the compression pad.

3. The apparatus according to either of claims 1 or 2, wherein the drive unit (312, 312', 312") is configured to receive at least one of power and control setting signals from a controller, and wherein the drive unit is configured to vary at least one of a pressure applied by the compression pad (350, 350', 350") to the breast, a duration of compression and decompression, and a heating level of the compression pad, and wherein the controller is coupled to the drive unit (312, 312', 312") with a wired or wireless coupling.

4. The apparatus according to any preceding claim, further comprising:
a plurality of beads (360, 360', 360") fixed to the compression pad (350, 350', 350") and through which the plurality of cables extend (352, 352', 352"), the beads being circumferentially spaced from one another around the outer surface of the compression pad, wherein the plurality of cables (352, 352', 352") are configured to slide in the beads (360, 360', 360").

5. The apparatus according to claim 1, wherein the transmission element includes a rack (374) connected to the first cable (352a', 352a") and a pinion gear (372) engaged with the rack (374) and driven by the motor (370), and wherein the second cable (352b', 352b"; 352c', 352c") is connected to a pull member (376, 378) and the rack (374) includes an engagement member (374a, 374b) configured to engage the pull member (376, 378) after a predetermined amount of relative translation between the rack and the pull member, and wherein the transmission element is configured to translate the cables (352, 352', 352") linearly relative to one another.

6. The apparatus according to claim 1, further comprising a cable routing unit (354, 354', 354") for routing at least one end of the plurality of cables from the compression pad (350, 350', 350") to the compression unit

7. The apparatus according to any previous claim, wherein the compression pad (350, 350', 350") is configured to connect to a bra that is configured to support the compression pad in engagement with the breast of the user.

8. The apparatus according to claim 3, further comprising a power supply to power at least one of the drive unit (312, 312', 312") and the controller.

9. The apparatus according to claim 1, wherein the transmission element is configured to translate the cables (352, 352', 352") linearly relative to one another.

10. A breastmilk expression system (300) comprising:
At least one breastmilk expression apparatus (302, 302') according to any preceding claim;
at least one strap (304) coupled to the at least one breast milk expression apparatus (302, 302') and configured to position the at least one breast milk expression apparatus on respective user's breasts; and
a controller configured to control the operation of the at least one breast milk expression apparatus (302, 302').

11. The system of claim 10, wherein the at least one strap comprises a bra (304), wherein the bra has an inner layer (320) and an outer layer (314) and at least a portion of the at least one breast milk expression apparatus (302, 302') is configured to be connected to the bra (304) between the inner and outer layer (320, 314).

12. The system of claim 11, wherein a portion of the inner layer (320) is formed as an inner bra cup (320a) and a portion of the outer layer (314) is formed as an outer bra cup (314a) configured to align with and fold over the inner bra cup (320a) in a first configuration and to move out of alignment with the inner bra cup (320a) in a second configuration, wherein outer bra cup (314a) is in the second configuration when the at least one breast milk expression apparatus (302, 302') is connected to the bra (304), wherein the inner bra cup (320a) defines a hole (322) that is configured to align with a hole in the breast milk expression apparatus when the breast milk expression apparatus (302, 302') is connected to the bra (304), wherein the hole (322) in the inner bra cup (320a) and the hole in the breast milk expression apparatus are configured to receive a tubular portion of a breastshield (310).

13. The system according to claim 11, wherein the compression pad (350, 350', 350") is configured to connect to the inner layer (320) of the bra,
wherein the compression pad includes a connector (326) configured to removably connect to another connector (324) secured to an inner layer (320) of the bra located about a base of a cup of the bra.

14. The system according to claim 13, wherein a portion of the inner layer (320) is formed as an inner bra cup (320a) and the other connector (324) secured to the inner layer of the bra extends about at least portion of a lower half of the base of the inner bra cup (320a).

15. The system according to claim 10, further comprising at least one of a heating pad, a cooling pad, and a medicated pad configured to couple to the at least one strap (304), wherein the heating pad, cooling pad, medicated pad, and breast milk expression apparatus are configured to interchangeably couple to the at least one strap (304).

## Patentansprüche

1. Eine Vorrichtung (302, 302') zur Gewinnung von Muttermilch, die Folgendes beinhaltet:
ein einzelnes Kompressionspolster (350, 350', 350"), das um eine Brust einer Benutzerin angeordnet werden kann und zur radialen Verlagerung relativ zu der Brust konfiguriert ist, wobei das Kompressionspolster eine konische äußere Oberfläche aufweist und sich in Längsrichtung von einem ersten Ende mit größerem Durchmesser zu einem zweiten Ende mit kleinerem Durchmesser erstreckt; und
eine Kompressionseinheit, die mit dem Kompressionspolster (350, 350', 350") gekoppelt ist, wobei die Kompressionseinheit eine Vielzahl von Seilen (352, 352', 352"), die um und durch das Kompressionspolster zusammengeschlossen sind, und eine Antriebseinheit (312, 312', 312"), die mit der Vielzahl von Seilen gekoppelt ist, beinhaltet, wobei die Antriebseinheit konfiguriert ist, um Spannung auf die Vielzahl von Seilen aufzubringen, um die Kompressionspolster (350, 350', 350") zyklisch radial einwärts zu verlagern, um eine Kompression der Brust zu bewirken, und der Brust im Anschluss an die Kompression der Brust zu gestatten, sich auszudehnen und zu dekomprimieren;
wobei die Vielzahl von Seilen ein erstes Seil (352a', 352a"), das sich in einer ersten Längsposition befindet, und ein zweites Seil (352b', 352b"; 352c', 352c"), das sich in einer zweiten Längsposition befindet, welche von der ersten Längsposition in Längsrichtung beabstandet ist und dichter an dem zweiten Ende liegt, umfasst;
wobei jedes Seil der Vielzahl von Seilen mit einer äußeren Oberfläche des Kompressionspolsters (350, 350', 350") gekoppelt ist und sich umlaufend um diese herum erstreckt, und wobei jedes Seil konfiguriert ist, um relativ zu dem Kompressionspolster zu gleiten; und
wobei die Antriebseinheit (312, 312', 312") einen Motor (370) und ein Getriebeelement (372, 374, 376, 378), das von dem Motor angetrieben wird, umfasst, wobei das Getriebeelement mit der Vielzahl von Seilen gekoppelt und konfiguriert ist, um die Seile sequenziell zu spannen, und **dadurch gekennzeichnet ist, dass** das Getriebeelement konfiguriert ist, um die Seile derart sequenziell zu spannen, dass Spannung für eine längere Dauer auf das erste Seil (352a', 352a") aufgebracht wird als auf das zweite Seil (352b', 352b"; 352c', 352c").

2. Vorrichtung gemäß Anspruch 1, wobei das Kompressionspolster (350, 350', 350") einen umlaufenden Kanal definiert, durch den sich die Vielzahl von Seilen (352, 352', 252") erstreckt, wobei der umlaufende Kanal eine relative Bewegung der Vielzahl von Seilen relativ zu dem Kompressionspolster gestattet.

3. Vorrichtung gemäß einem der Ansprüche 1 oder 2, wobei die Antriebseinheit (312, 312', 312") konfiguriert ist, um mindestens eines von Leistung und Steuerungseinstellungssignalen von einer Steuereinheit zu empfangen, und wobei die Antriebseinheit konfiguriert ist, um mindestens eines von einem Druck, der durch das Kompressionspolster (350, 350', 350") auf die Brust aufgebracht wird, einer Dauer der Kompression und Dekompression und einem Heizniveau des Kompressionspolsters zu variieren, und wobei die Steuereinheit mit einer drahtgebundenen oder drahtlosen Kopplung mit der Antriebseinheit (312, 312', 312") gekoppelt ist.

4. Vorrichtung gemäß einem der vorhergehenden Ansprüche, die ferner Folgendes beinhaltet:
eine Vielzahl von Perlen (360, 360', 360"), die an dem Kompressionspolster (350, 350', 350") fixiert sind und durch die sich die Vielzahl von Seilen (352, 352', 352") erstreckt, wobei die Perlen um die äußere Oberfläche des Kompressionspolsters herum umlaufend voneinander beabstandet sind, wobei die Vielzahl von Seilen (352, 352', 352") konfiguriert ist, um in den Perlen (360, 360', 360") zu gleiten.

5. Vorrichtung gemäß Anspruch 1, wobei das Getriebeelement eine Zahnstange (374), die mit dem ersten Seil (352a', 352a") verbunden ist, und ein Ritzel (372), das mit der Zahnstange (374) in Eingriff steht und von dem Motor (370) angetrieben wird, umfasst, und wobei das zweite Seil (352b', 352b"; 352c', 352c") mit einem Zugteil (376, 378) verbunden ist und die Zahnstange (374) ein Eingriffsteil (374a, 374b) umfasst, das konfiguriert ist, um nach einem vorgegebenen Betrag relativer Verschiebung zwischen der Zahnstange und dem Zugteil in das Zugteil (376, 378) einzugreifen, und wobei das Getriebeelement konfiguriert ist, um die Seile (352, 352', 352") relativ zueinander linear zu verschieben.

6. Vorrichtung gemäß Anspruch 1, die ferner eine Seilführungseinheit (354, 354', 354") zum Führen mindestens eines Endes der Vielzahl von Seilen von dem Kompressionspolster (350, 350', 350") zu der Kompressionseinheit beinhaltet.

7. Vorrichtung gemäß einem der vorhergehenden Ansprüche, wobei das Kompressionspolster (350, 350', 350") konfiguriert ist, um mit einem Büstenhalter verbunden zu werden, der konfiguriert ist, um das Kompressionspolster im Eingriff mit der Brust der Benutzerin zu stützen.

8. Vorrichtung gemäß Anspruch 3, die ferner eine Leistungsversorgung zum Versorgen von mindestens einem von der Antriebseinheit (312, 312', 312") und der Steuereinheit mit Leistung beinhaltet.

9. Vorrichtung gemäß Anspruch 1, wobei das Getriebeelement konfiguriert ist, um die Seile (352, 352', 352") relativ zueinander linear zu verschieben.

10. Ein System (300) zur Gewinnung von Muttermilch, das Folgendes beinhaltet:
mindestens eine Vorrichtung (302, 302') zur Gewinnung von Muttermilch gemäß einem der vorhergehenden Ansprüche;
mindestens einen Riemen (304), der mit der mindestens einen Vorrichtung (302, 302') zur Gewinnung von Muttermilch gekoppelt ist und konfiguriert ist, um die mindestens eine Vorrichtung zur Gewinnung von Muttermilch auf jeweiligen Brüsten der Benutzerin zu positionieren; und
eine Steuereinheit, die konfiguriert ist, um den Betrieb der mindestens einen Vorrichtung (302, 302') zur Gewinnung von Muttermilch zu steuern.

11. System gemäß Anspruch 10, wobei der mindestens eine Riemen einen Büstenhalter (304) beinhaltet, wobei der Büstenhalter eine innere Schicht (320) und eine äußere Schicht (314) aufweist und mindestens ein Abschnitt der mindestens einen Vorrichtung (302, 302') zur Gewinnung von Muttermilch konfiguriert ist, um mit dem Büstenhalter (304) zwischen der inneren und der äußeren Schicht (320, 314) verbunden zu werden.

12. System gemäß Anspruch 11, wobei ein Abschnitt der inneren Schicht (320) als ein inneres Büstenhalter-Körbchen (320a) gebildet ist und ein Abschnitt der äußeren Schicht (314) als ein äußeres Büstenhalter-Körbchen (314a) gebildet ist, das konfiguriert ist, um sich in einer ersten Konfiguration mit dem inneren Büstenhalter-Körbchen (320a) zu decken und darüber gefaltet zu sein und sich in einer zweiten Konfiguration aus der Deckung mit dem inneren Büstenhalter-Körbchen (320a) zu bewegen, wobei das äußere Büstenhalter-Körbchen (314a) in der zweiten Konfiguration ist, wenn die mindestens eine Vorrichtung (302, 302') zur Gewinnung von Muttermilch mit dem Büstenhalter (304) verbunden ist, wobei das innere Büstenhalter-Körbchen (320a) ein Loch (322) definiert, das konfiguriert ist, um sich mit einem Loch in der Vorrichtung zur Gewinnung von Muttermilch zu decken, wenn die Vorrichtung (302, 302') zur Gewinnung von Muttermilch mit dem Büstenhalter (304) verbunden ist, wobei das Loch (322) in dem inneren Büstenhalter-Körbchen (320a) und das Loch in der Vorrichtung zur Gewinnung von Muttermilch konfiguriert sind, um einen röhrenförmigen Abschnitt einer Brusthaube (310) aufzunehmen.

13. System gemäß Anspruch 11, wobei das Kompressionspolster (350, 350', 350") konfiguriert ist, um mit der inneren Schicht (320) des Büstenhalters verbunden zu werden,
wobei das Kompressionspolster einen Verbinder (326) umfasst, der konfiguriert ist, um abnehmbar mit einem weiteren Verbinder (324) verbunden zu werden, der an einer inneren Schicht (320) des Büstenhalters befestigt ist, die um eine Basis eines Körbchens des Büstenhalters befindlich ist.

14. System gemäß Anspruch 13, wobei ein Abschnitt der inneren Schicht (320) als ein inneres Büstenhalter-Körbchen (320a) gebildet ist und sich der weitere Verbinder (324), der an der inneren Schicht des Büstenhalters befestigt ist, um mindestens einen Abschnitt einer unteren Hälfte der Basis des inneren Büstenhalter-Körbchens (320a) herum erstreckt.

15. System gemäß Anspruch 10, das ferner mindestens eines von einem Heizpolster, einem Kühlpolster und einem Arzneistoff enthaltendem Polster beinhaltet, die konfiguriert sind, um mit dem mindestens einen Riemen (304) gekoppelt zu werden, wobei das Heizpolster, das Kühlpolster, das Arzneistoff enthaltende Polster und die Vorrichtung zur Gewinnung von Muttermilch konfiguriert sind, um austauschbar mit dem mindestens einen Riemen (304) gekoppelt zu werden.

## Revendications

1. Un appareil d'expression de lait maternel (302, 302') comprenant :
un unique coussinet de compression (350, 350', 350") pouvant être disposé autour d'un sein d'une utilisatrice et configuré pour un déplacement radial par rapport audit sein, le coussinet de compression présentant une surface externe conique et s'étendant longitudinalement depuis une première extrémité de plus grand diamètre jusqu'à une deuxième extrémité de plus petit diamètre ; et
une unité de compression couplée au coussinet de compression (350, 350', 350"), l'unité de compression comprenant une pluralité de câbles (352, 352', 352") en laçage autour et au travers du coussinet de compression, et une unité d'entraînement (312, 312', 312") couplée à la pluralité de câbles, l'unité d'entraînement étant configurée pour appliquer une tension à la pluralité de câbles afin de déplacer de façon cyclique le coussinet de compression (350, 350', 350") radialement vers l'intérieur pour provoquer la compression du sein et, après la compression du sein, permettre au sein de se dilater et de décompresser ;
dans lequel la pluralité de câbles inclut un premier câble (352a', 352a") situé au niveau d'un première position longitudinale et un deuxième câble (352b', 352b" ; 352c', 352c") situé au niveau d'une deuxième position longitudinale qui est espacée longitudinalement de la première position longitudinale et plus près de la deuxième extrémité ;
dans lequel chaque câble de la pluralité de câbles est couplé à une surface externe du coussinet de compression (350, 350', 350") et s'étend circonférentiellement autour de celle-ci, et dans lequel chaque câble est configuré pour coulisser par rapport au coussinet de compression ; et
dans lequel l'unité d'entraînement (312, 312', 312") inclut un moteur (370) et un élément de transmission (372, 374, 376, 378) entraîné par le moteur, l'élément de transmission étant couplé à la pluralité de câbles et configuré pour mettre séquentiellement les câbles en tension et **caractérisé en ce que** l'élément de transmission est configuré pour mettre séquentiellement les câbles en tension de telle sorte que la tension sur le premier câble (352a', 352a") est appliquée pendant une durée plus longue que celle sur le deuxième câble (352b', 352b" ; 352c', 352c").

2. L'appareil selon la revendication 1, dans lequel le coussinet de compression (350, 350', 350") définit un canal circonférentiel à travers lequel s'étend la pluralité de câbles (352, 352', 352"), le canal circonférentiel permettant le mouvement relatif de la pluralité de câbles par rapport au coussinet de compression.

3. L'appareil selon soit la revendication 1, soit la revendication 2, dans lequel l'unité d'entraînement (312, 312', 312") est configurée pour recevoir au moins un signal parmi des signaux de réglage de puissance et de commande en provenance d'un dispositif de commande, et dans lequel l'unité d'entraînement est configurée pour faire varier au moins une caractéristique parmi une pression appliquée par le coussinet de compression (350, 350', 350") au sein, une durée de compression et de décompression, et un niveau de chauffage du coussinet de compression, et dans lequel le dispositif de commande est couplé à l'unité d'entraînement (312, 312', 312") grâce à un couplage filaire ou sans fil.

4. L'appareil selon n'importe quelle revendication précédente, comprenant en outre :
une pluralité de billes (360, 360', 360") fixées au coussinet de compression (350, 350', 350") et au travers desquelles s'étend la pluralité de câbles (352, 352', 352"), les billes étant espacées circonférentiellement les unes des autres autour de la surface externe du coussinet de compression, la pluralité de câbles (352, 352', 352") étant configurés pour coulisser dans les billes (360, 360', 360").

5. L'appareil selon la revendication 1, dans lequel l'élément de transmission inclut une crémaillère (374) raccordée au premier câble (352a', 352a") et une roue d'engrenage (372) en prise avec la crémaillère (374) et entraînée par le moteur (370), et dans lequel le deuxième câble (352b', 352b" ; 352c', 352c") est raccordé à un organe de traction (376, 378) et la crémaillère (374) inclut un organe de mise en prise (374a, 374b) configuré pour mettre en prise l'organe de traction (376, 378) après une quantité prédéterminée de translation relative entre la crémaillère et l'élément de traction, et dans lequel l'élément de transmission est configuré pour faire translater les câbles (352, 352', 352") linéairement les uns par rapport aux autres.

6. L'appareil selon la revendication 1, comprenant en outre une unité de routage de câble (354, 354', 354") destinée à router au moins une extrémité de la pluralité de câbles du coussinet de compression (350, 350', 350") vers l'unité de compression.

7. L'appareil selon n'importe quelle revendication précédente, dans lequel le coussinet de compression (350, 350', 350") est configuré pour être raccordé à un soutien-gorge qui est configuré pour soutenir le coussinet de compression dans sa mise en prise avec le sein de l'utilisatrice.

8. L'appareil selon la revendication 3, comprenant en outre un bloc d'alimentation pour alimenter au moins un élément parmi l'unité d'entraînement (312, 312', 312") et le dispositif de commande.

9. L'appareil selon la revendication 1, dans lequel l'élément de transmission est configuré pour faire translater les câbles (352, 352', 352") linéairement les uns par rapport aux autres.

10. Un système de d'expression de lait maternel (300) comprenant :
au moins un appareil d'expression de lait maternel (302, 302') selon n'importe quelle revendication précédente ;
au moins une bretelle (304) couplée à l'au moins un appareil d'expression de lait maternel (302, 302') et configurée pour positionner l'au moins un appareil d'expression de lait maternel sur les seins respectifs d'une utilisatrice ; et
un dispositif de commande configuré pour commander le fonctionnement de l'au moins un appareil d'expression de lait maternel (302, 302').

11. Le système de la revendication 10, dans lequel l'au moins une bretelle comprend un soutien-gorge (304), le soutien-gorge présentant une couche interne (320) et une couche externe (314) et au moins une partie de l'au moins un appareil d'expression de lait maternel (302, 302') est configurée pour être raccordée au soutien-gorge (304) entre la couche interne et externe (320, 314).

12. Le système de la revendication 11, dans lequel une partie de la couche interne (320) est formée comme un bonnet interne (320a) de soutien-gorge et une partie de la couche externe (314) est formée comme un bonnet externe (314a) de soutien-gorge configuré pour être en alignement avec le bonnet interne (320a) de soutien-gorge et se replier par-dessus celui-ci dans une première configuration et se mouvoir hors d'alignement avec le bonnet interne (320a) de soutien-gorge dans une deuxième configuration, le bonnet externe (314a) de soutien-gorge étant dans la deuxième configuration lorsque l'au moins un appareil d'expression de lait maternel (302, 302') est raccordé au soutien-gorge (304), le bonnet interne (320a) de soutien-gorge définissant un trou (322) qui est configuré pour s'aligner avec un trou dans l'appareil d'expression de lait maternel lorsque l'appareil d'expression de lait maternel (302, 302') est raccordé au soutien-gorge (304), le trou (322) dans le bonnet interne (320a) de soutien-gorge et le trou dans l'appareil d'expression de lait maternel étant configurés pour recevoir une partie tubulaire d'une téterelle (310).

13. Le système selon la revendication 11, dans lequel le coussinet de compression (350, 350', 350") est configuré pour se raccorder à la couche interne (320) du soutien-gorge, dans lequel le coussinet de compression inclut un connecteur (326) configuré pour se raccorder de façon amovible à un autre connecteur (324) assujetti à une couche interne (320) du soutien-gorge, situé autour d'une base d'un bonnet du soutien-gorge.

14. Le système selon la revendication 13, dans lequel une partie de la couche interne (320) est formée comme un bonnet interne (320a) de soutien-gorge et l'autre connecteur (324) assujetti à la couche interne du soutien-gorge s'étend autour d'au moins une partie d'une moitié inférieure de la base du bonnet interne (320a) de soutien-gorge.

15. Le système selon la revendication 10, comprenant en outre au moins un coussinet parmi un coussinet chauffant, un coussinet refroidissant, et un coussinet traitant, configuré pour se coupler à l'au moins une bretelle (304), le coussinet chauffant, le coussinet refroidissant, le coussinet médicamenteux, et l'appareil d'expression de lait maternel étant configurés pour se coupler de façon interchangeable à l'au moins une bretelle (304).
